# EUROPÄISCHE PATENTANMELDUNG

(11) **EP 0 849 278 A1**
(43) Veröffentlichungstag der Anmeldung: **24.06.1998**
(21) Anmeldenummer: 97122298.9
(22) Anmeldetag: 17.12.1997
(51) Int. Cl.: C07K 19/00, G01N 33/533

(54) **Niedrig vernetzte Photoprotein-Konjugate**

(30) Priorität: 17.12.1996 DE 19652576
(71) Anmelder: Boehringer Mannheim GmbH, 68305 Mannheim-Waldhof (DE)
(72) Erfinder: Weindel, Kurt, Dr., 82407 Wielenbach (DE); Kanne, Beata, 82061 Neuried (DE); Seidenschwarz, Erika, 82343 Poecking (DE); Stults, Nancy, Dr., Cary, NC 27513 (US); Smith, David F., Dr., Athens, GA 30606 (US)
(74) Vertreter: Weiss, Wolfgang, Dipl.-Chem. Dr.

(57) **Zusammenfassung**

Die Erfindung betrifft eine Konjugatzusammensetzung aus einem Photoprotein und einem Bindepartner, die überwiegend Konjugatmoleküle enthält, in denen das Photoprotein und der Bindepartner in singulärer Anzahl kovalent miteinander verknüpft sind und deren Verwendung als Nachweisreagenz.

## Beschreibung

Die Erfindung betrifft neue Konjugatzusammensetzungen aus einem Photoprotein und einem Bindepartner und Verfahren zu deren Herstellung. Weiterhin wird ein Verfahren zum Nachweis eines Analyten in einer Probe durch Kontaktieren der Probe mit der erfindungsgemäßen Konjugatzusammensetzung als Analyt-spezifisches oder universales Nachweisreagenz sowie ein Reagenzienkit, der neben anderen Testkomponenten eine erfindungsgemäße Konjugatzusammensetzung als Nachweisreagenz enthält, offenbart.

Unter dem Begriff Rezeptor-Ligand-Bindungsassay werden alle Typen von Testverfahren verstanden, die auf der biospezifischen Wechselwirkung zweier molekularer Einheiten beruhen. Für solche Testverfahren werden häufig als Nachweisreagenzien Konjugate eingesetzt, die eine biospezifisch reaktive Komponente und eine signalgebende Komponente enthalten. Zur Optimierung des Testverfahrens werden dabei ein breiter dynamischer Meßbereich, eine hohe Empfindlichkeit, eine gute Präzision und hohe Signal-Rausch-Verhältnisse angestrebt. Eine wichtige Voraussetzung dabei ist die Retention einer hohen biospezifischen Reaktivität der einen Komponente und eine hohe Markierungsaktivität der anderen signalgebenden Komponente im Konjugat.

Zu diesem Zweck wurden verschiedene Strategien entwickelt, die auf eine Multiplizität der spezifischen Reaktivität oder Markierungsaktivität oder beider Aktivitäten im Konjugat hinauslaufen. Als Beispiele genannt seien hier die Verstärkung der Chemilumineszenz von Acridiniumesterkonjugaten durch Verkapselung von Acridiniumestermolekülen in funktionalisierten Liposomen und Kopplung derselben an die biospezifische Komponente (Law et al. , J. Bioluminescence and Chemiluminescence 4 (1989), 88 bis 98; US-PS 5,449,556), die multiple kovalente Vernetzung (Oligomerisierung) von biospezifischen Komponenten und Markierungskomponenten (WO 96/23226), die Vielfachmarkierung von biospezifischen Komponenten mit Europium-Chelaten, z. B. 3 bis 4 Markierungsgruppen pro Antikörper mittels Diazophenyl-EDTA-Europium bzw. bis zu 50 Markierungsgruppen pro Antikörper mittels p-Isothiocyanatobenzyl-EDTA-Europium (Hemmilä et al., Anal. Biochem. 137 (1984), 335 - 343) oder ca. 20 Markierungsgruppen pro Oligonucleotid mittels p-Isothiocyanatobenzyl-EDTA-Europium (DELFIA® Research Products, Sensitve measurenments without radioactivity; Wallac Oy Turku/Finnland, Informations-Broschüre Nr. 1244-1202-03).

Die Verwendung von Lumineszenzmarkierungen als Nachweissystem hat gegenüber anderen Nachweissystemen, z.B. radioaktiven Markierungsgruppen, den Vorteil einer höheren Sensitivität. Ein Beispiel für Lumineszenzmarkierungen, die sich für den Einsatz in Nachweisverfahren eignen, sind Photoproteine, z.B. Ca-aktivierbare Photoproteine der Aequorin-Familie.

Aequorin ist ein Photoprotein bestehend aus Apo-Aequorin, der katalytisch aktiven Proteinkomponente, Coelenterazin, der oxidierbaren Luminophorkomponente sowie molekularem, wahrscheinlich als Hydroperoxid an das Protein gebundenem Sauerstoff, dem Oxidationsmittel. Durch Zugabe von Ca²⁺-Ionen (Trigger) kommt es zu einer Lumineszenzreaktion zwischen den einzelnen Komponenten des Reaktionskomplexes. Die Aequorin-Biolumineszenz zeichnet sich aus durch außergewöhnlich hohe Photonenausbeute (≥ 25%) und Schnelligkeit (< 5 sec.) (vgl. Blinks, J.Pharmacological Reviews 28 (1976), 1-93).

Ein Nachteil von Aequorin und verwandten Photoproteinen ist jedoch, daß sie äußerst empfindlich gegenüber jeglicher chemischer Modifikation sind. Die Folgen sind üblicherweise Verlust der katalytischen Aktivität und/oder vorzeitige Entladung ohne Triggerzugabe.

US-A-5,486,455 beschreibt eine schonende Modifizierung von Photoproteinen durch Einführung zusätzlicher SH-Gruppen. An diese SH-Gruppen können über geeignete reaktive Gruppen, z.B. Maleimidgruppen, Bindepartner wie etwa Proteine, Nucleinsäuren oder Haptene gekoppelt werden. Auf diese Weise ist die Herstellung von Photoprotein-Bindepartner-Konjugaten unter hohem Erhalt der Photoprotein-Aktivität möglich. Die Aufreinigung der in US-A-5,486,455 offenbarten Konjugate erfolgt dadurch, daß die Reaktionsprodukte aus der Kopplungsreaktion des Bindepartners und des Photoproteins nach Abtrennung nichtreagierter Ausgangsmaterialien gepoolt werden. Eine Subfraktionierung der Produkte der Kopplungsreaktion wird nicht beschrieben.

Obwohl die Photoprotein-Bindepartner-Konjugate gemäß US-A-5,486,455 sich als Nachweisreagenzien in Testverfahren bewährt haben, besteht ein Bedarf nach Nachweisreagenzien mit verbesserter Leistung, z.B. hinsichtlich der Meßbereichdynamik und der analytischen Sensitivität.

Überraschenderweise wurde festgestellt, daß in den verschiedensten Typen von Rezeptor-Ligand-Bindungsassays gerade der Einsatz von niedermolekularen Photoprotein-Bindepartner-Konjugatzusammensetzungen zu einer Verbesserung der Testleistung führt. Diese niedermolekularen Konjugatzusammensetzungen sind durch Subfraktionierung der bei der Kopplungsreaktion von Photoprotein und Bindepartner entstehenden Reaktionsprodukte oder/und durch Verwendung spezieller Reaktionspartner bzw. Kopplungsstöchiometrien erhältlich. Besonders bevorzugt werden in der Praxis beide Maßnahmen kombiniert.

Ein Gegenstand der vorliegenden Erfindung ist somit eine niedermolekulare Konjugatzusammensetzung aus einem Photoprotein und einem Bindepartner, die überwiegend Konjugatmoleküle enthält, in denen das Photoprotein und der Bindepartner in singulärer Anzahl miteinander kovalent verknüpft sind. Die erfindungsgemäße Konjugatzusammensetzung soll günstigerweise weitgehend frei von hochmolekularen Aggregaten sein, in denen mehrere Moleküle des Bindepartners oder/und des Photoproteins miteinander verknüpft sind. Vorzugsweise enthält die Konjugatzusammensetzung einen Anteil von mindestens 70% an niedermolekularen Konjugaten aus Photoprotein und Bindereagenz, besonders bevorzugt einen Anteil von mindestens 80%, am meisten bevorzugt einen Anteil von mindestens 90% bezogen auf die Gesamtheit der Konjugatmoleküle in der Zusammensetzung.

Weiterhin bevorzugt ist, daß das Molverhältnis von Photoprotein zu Bindepartner in der Konjugatzusammensetzung im Bereich von 1:0,8 - 1:2, insbesondere im Bereich von 1:1 - 1:1,5 liegt. Am meisten bevorzugt beträgt das Molverhältnis von Photoprotein zu Bindepartner etwa 1:1.

Das Photoprotein ist vorzugsweise ein Ca-aktivierbares Photoprotein. Unter diesem Begriff wird eine Familie von Lumineszenz-fähigen Proteinen zusammengefaßt, die wie Aequorin einen Reaktionskomplex aus Protein (Apo-Photoprotein), einem organisch-chemischen Substrat, welches fest, aber nicht kovalent gebunden ist und den Photoemitter darstellt, (Luminophor) und ebenfalls fest fixiertem molekularem Sauerstoff, der erforderlich ist für die Initiierung der Lumineszenzreaktion (Oxidans) bilden. Die Lumineszenzreaktion wird durch Bindung von Ca²⁺-Ionen an das Apo-Photoprotein ausgelöst und ist vom Sauerstoff aus der Umgebung unabhängig.

Besonders bevorzugt weisen Ca-aktivierbare Photoproteine folgende gemeinsame Merkmale auf:
a) physiko-chemische Aspekte:
   - fertig ausgebildeter Reaktionskomplex aus einem proteinösen Katalysator (=Apo-Photoprotein), einem organischchemischen Substrat, welches fest, aber nicht kovalent gebunden ist und den eigentlichen Emitter darstellt (=Luminophor), und ebenfalls fest fixiertem molekularem Sauerstoff (vermutlich kovalent protein-gebunden als Hydroperoxid), der erforderlich ist für die Initiierung der Lumineszenzreaktion (=Oxidans)
   - Unterscheidung von Enzymsystemen durch Gebundensein aller für die Lumineszenzreaktion notwendigen Komponenten
   - relative Molmasse im Bereich von ca. 22000 Dalton
   - "Coelenterazin" ([2-(p-Hydroxybenzyl)-6-(p-hydroxyphenyl)-8-benzyl-7-hydroimidazopyrazin-3-on] als natürliches Luminophor
   - Emissionspeakwellenlänge (λmax) im Blaubereich (ca. 470 nm)
   - Auslösung der Lumineszenzreaktion durch Bindung von 2-3 Ca ²⁺-Ionen an das Apo-Photoprotein
   - Vorhandensein von Ca²⁺ -Bindedomänen im Apo-Photoprotein in Form von EF-Hand- (=Helix-Schleife-Helix-)strukturen
   - Unabhängigkeit der Lumineszenzreaktion von Sauerstoff aus der Umgebung, d.h. das komplette Photoprotein luminesziert in Gegenwart wie auch in Abwesenheit von O₂ in der umgebenden Atmosphäre
   - Photoprotein-Reaktion verläuft in Form einer Blitzkinetik, d.h. konzentrierte Lichtemission als Folge von Ca²⁺-Ionen-Zugabe (siehe hierzu Blinks J.R., et al., Pharmacological Reviews 28/1, 1-93, 1976)
b) molekularbiologische Aspekte
   - funktionstaugliche Apo-Photoproteine mit einer Länge von im allgemeinen 189-196 Aminosäuren
   - hohe Übereinstimmung der Nucleobasen- und Aminosäuresequenzen (≥ 60 % Homologie) der einzelnen Verteter der Familie der Ca²⁺-aktivierbaren Photoproteine.

Beispiele für geeignete Ca-aktivierbare Photoproteine sind Aequorin, Obelin, Clytin, Mitrocomin, Berovin, Mnemiopsin und Thalassicolin. Vorzugsweise wird Aequorin verwendet.

Die Ca-aktivierbaren Photoproteine können als native Proteine, die aus ihren Ursprungsorganismen isoliert wurden, oder als rekombinante Proteine, z.B. aus E.coli eingesetzt werden. Die Verwendung rekombinanter Photoproteine ist bevorzugt. Aequorin stammt beispielsweise aus dem Organismus Aequorea victoria. Die Klonierung und die rekombinante Herstellung von Aequorin in E.coli ist von Prasher et al., Biochem. Biophys. Res. Comm. 126/3 (1985), 1259-1268, Prasher et al., Meth. Enzymol. 133 (1986), 288-299, Prasher et al., Biochemistry 26 (1987), 1326-1332, Cormier et al., Photochemistry and Photobiology 49/4 (1989), 509-512 und Stults et al., Biochemistry 31 (1992), 1433-1442 beschrieben. Die Klonierung, Expression und Sequenz des aus dem Organismus Obelia longissima stammenden Photoproteins Obelin sind bei Illarionov et al., J.Biolumineszenz und Chemolumineszenz 8 (1993), VII. International Symposium-Abstracts 88 und Gene 153 (1995), 273-274 beschrieben. Klonierung, Expression und Sequenzanalyse der Photoproteine Clytin und Mitrocomin sind bei Inouye und Tsuji (FEBS 315 (1993), 343-346) und Fagan et al. (FEBS 333 (1993), 301-305) beschrieben. Weitere Mitglieder der Aequorin-Familie sind die Photoproteine Berovin und Mnemiopsin (Ward und Seliger, Biochemistry 13 (1974), 1491-1499 und 1500-1510) und Thalassicolin (Campbell et al., Application of the photoprotein obelin to the measurement of Ca²⁺ in cells. In: Bioluminescence and Chemiluminescence, Hrsg. DeLuca M. A. & McElroy, W. D., p. 601-607 (1981), Academic Press, New York). Auf die in den oben genannten Literaturstellen enthaltene Offenbarung zur Gewinnung von Ca-aktivierbaren Photoproteinen wird hiermit Bezug genommen.

Besonders bevorzugt ist das Photoprotein ein Sulfhydryl-aktiviertes Photoprotein, welches über die Sulhydrylgruppe mit dem Bindereagenz gekoppelt ist. Die Einführung von Sulfhydrylgruppen in Photoproteine kann beispielsweise durch chemische Modifikation mit geeigneten Reagenzien wie etwa N-Succinimidyl-(S-acetyl) thioacetat (SATA), N-Succinimidyl-(S-acetyl)thiopropionat (SATP), S-Acetylmercaptosuccinanhydrid (SAMSA) und insbesondere 2-Iminothiolan (Traut's-Reagenz) und gegebenenfalls anschließende Schutzgruppenabspaltung, z. B. mit einem Reduktionsmittel wie etwa Hydroxylamin, erfolgen. Diese chemische Modifikation ist in US-A-5,486,455 beschrieben. Auf dieses Verfahren wird ausdrücklich Bezug genommen.

Der mit dem Photoprotein gekoppelte Bindepartner kann eine beliebige biologische Substanz sein. Bevorzugt ist eine biologische Substanz mit einem Molekulargewicht von ≥ 4 kD, da bei solchen Substanzen die Vorteile von niedermolekularen Konjugaten gegenüber oligomeren Konjugaten besonders stark ausgeprägt sind.

Der Bindepartner ist eine Substanz, die in einem Nachweisverfahren direkt oder indirekt an einen zu bestimmenden Analyten binden oder mit diesem um die Bindung an einen Rezeptor kompetieren kann. Bevorzugt ist der Bindeparter ein Nicht-Hapten mit einem Molekulargewicht ≥ 4 kDa. Beispiele von Bindepartnern sind Polypeptide wie etwa Streptavidin, Avidin, Enzyme, Glycoproteine, Peptide, Rezeptoren und insbesondere Immunglobuline oder Immunglobulinfragmente. Weiterhin kann der Bindepartner ein Lectin, Hormon, Arzneimittel etc. sein. In einer weiteren bevorzugten Ausführungsform der vorliegenden Erfindung ist der Bindepartner eine Nucleinsäure, z.B. RNA, DNA oder ein Nucleinsäureanalogon wie eine peptidische Nucleinsäure (PNA).

Bevorzugt weist der Bindepartner mindestens eine Aminogruppe auf, die nach einer chemischen Modifizierung mit der Sulfhydrylgruppe des Photoproteins reagieren kann. Beispiele für geeignete reaktive Gruppen, die durch chemische Modifizierung in den Bindepartner eingeführt werden können, sind α-Halogencarbonylgruppen und insbesondere Maleimidgruppen. Die Einführung von Maleimidgruppen kann beispielsweise durch bifunktionelle Reagenzien erfolgen wie etwa Succinimidyl-4-(N-maleimidomethyl)cyclohexan (SMCC), Sulfosuccinimidyl-4-(N-maleimidomethyl)cyclohexan-1-carboxylat(Sulfo-SMCC), N-Maleimidobenzoyl-N-hydroxysuccinimidester(MBS) bzw. dem sulfonierten Derivat von MBS (Sulfo-MBS), Succinimidyl-4-(p-maleimidophenyl)butyrat (SMPB) bzw. dem sulfonierten Derivat davon (Sulfo-SMPB), Maleimidohexanoyl-N-hydroxy-succinimidester (MHS), N¹-[8-(N.Succinimidyl) suberylamido] N⁸-[maleinimido]-1,8-diamino-3,6-dioxaoctan (SS-MADOO) und Bis-maleimidomethylether (BMME). Besonders bevorzugt wird MHS verwendet.

Um eine erfindungsgemäße Konjugatzusammensetzung zu erhalten, die einen möglichst hohen Anteil an niedermolekularen singulären verknüpften Komponenten enthält, kann man ein Sulfhydrylaktiviertes Photoprotein einsetzen, welches durchschnittlich weniger als zwei und insbesondere weniger als 1,6 zusätzliche Sulfhydrylgruppen pro Mol Photoprotein aufweist. Weiterhin kann man einen Bindepartner einsetzen, welcher durchschnittlich weniger als 1,5 reaktive Gruppen aufweist, die mit dem Photoprotein reagieren können. Dabei ist anzumerken, daß erfindungsgemäße Konjugate auch nach dem in DE-A-44 38 660 beschriebenen Kopplungsverfahren hergestellt werden können, wobei der Bindepartner vorzugsweise über eine Carbonsäurefunktion an eine Aminogruppe des Photoproteins gekoppelt wird.

In einer besonders bevorzugten Ausführungsform der vorliegenden Erfindung setzt man einen Bindepartner ein, der eine singuläre und vorzugsweise positional lokalisierte reaktive Gruppe aufweist und über diese Gruppe mit dem Photoprotein gekoppelt werden kann. So erhält man ein Konjugat, welches zumindest auf Seite des Bindepartners eine exakt definierte Stöchiometrie aufweist. Auf diese Weise können beispielsweise Konjugate von Photoproteinen sowohl mit immunreaktiven Polypeptiden als auch mit Nucleotiden bzw. Nucleotidanaloga hergestellt werden.

Konjugate von Photoproteinen mit immunreaktiven Substanzen lassen sich beispielsweise gewinnen durch Spaltung von Mausγ1-Immunglobulinen mittels Pepsin, nachfolgende reduktive Spaltung der F(ab')₂-Fragmente mit Sulfhydrylreagenzien wie etwa Mercaptoethanol oder Cysteamin und Aktivierung der freigesetzten SH-Gruppe im resultierenden Fab'-Fragment. Diese Aktivierung kann durch Umsetzen mit einem Reagenz erfolgen, das eine Aminogruppe, bevorzugt eine primäre Aminogruppe trägt wie z.B. Jodacetamid oder bevorzugt N-Jodethyltrifluoracetamid, aus dem aus alkalischer Abspaltung von Trifluoressigsäure eine primäre NH₂-Gruppe freigesetzt wird, die mit Reagenzien wie MHS oder SMCC durch Einführung einer Maleinimidgruppe aktiviert werden kann. Andererseits kann auch eine direkte SH-Derivatisierung mit einem Bis-MH-Reagenz erfolgen. Aus Nicht-Maus-γ1-IgG-Immunglobulinen, die mehrere Disulfidbrücken zwischen den beiden schweren Ketten im Bereich der Hingeregion aufweisen, können positional definierte 1:1 Konjugate hergestellt werden mittels Umsetzung mit N-Jodethyltrifluoracetamid und nachfolgender Reaktion mit z.B. MHS in einer Stöchiometrie, die statistisch nur eine MH:SH-Kopplungsstelle bevorzugt dort erzeugt, wo die primäre Alkylaminofunktion in erhöhter Dichte eingeführt wurde. Ein weiterer Vorteil dieses Verfahrens ist, daß die Kopplung auf der der Antigen-Bindedomäne abgewandten Seite erfolgt und zu einer erhöhten Retention der Immunoreaktivität führt.

Photoprotein-Konjugate von hybridisierungsfähigen Substanzen lassen sich herstellen über gezielte Einfachfunktionalisierung von Nucleinsäuren, z.B. während der chemischen Synthese durch entsprechende Modifikation der Nucleobase, des Zuckers oder der Phosphatgruppe. Beispielsweise kann eine Funktionalisierung durch Einbau eines N-Trifluoracetamido-(3-oxa)-pentyl [N,N-diisopropyl-methyl]phosphoramidits am 5'-Ende erfolgen, aus welchem wiederum per Baseneinwirkung ein primäres terminales Amin freigesetzt werden kann. Alternativ dazu kann 5'-terminal auch direkt ein Alkylaminodeoxyzuckernucleosid eingebaut werden oder innerhalb der Sequenz Aminoalkylphosphoramidite zur Internucleosid-Funktionalisierung bzw. Nucleotide mit Alkylamino-derivatisierter Nucleobase für eine basenspezifische Funktionalisierung. Nach Aktivierung der Aminogruppe beispielsweise mit MHS liegt dann jeweils eine Nucleinsäure oder ein Nucleinsäureanalogon vor, das mit einem niedrig aktivierten SH-Photoproteins zur 1:1 Kopplung gebracht werden kann.

Ein weiterer Gegenstand der vorliegenden Erfindung ist ein Verfahren zur Herstellung einer erfindungsgemäßen Konjugatzusammensetzung, wobei man das Photoprotein und den Bindepartner unter Bedinungen in Kontakt bringt, bei denen kovalente Kopplungsprodukte zwischen Photoprotein und Bindepartner gebildet werden und eine niedermolekulare Fraktion der Kopplungsprodukte von einer höhermolekularen Fraktion der Kopplungsprodukte und nicht reagierten Ausgangsmaterialien auftrennt.

Vorzugsweise erfolgt die Aufreinigung der gewünschten niedermolekularen Fraktion derart, daß zunächst eine Ionenaustauschchromatographie durchgeführt wird, wobei aus dem Reaktionsansatz der freie Bindepartner abgetrennt wird. In einem zweiten Aufreinigungsschritt wird dann das nicht reagierte Photoprotein vom Konjugat aufgrund der unterschiedlichen Größe mittels Gelfiltrationchromatographie abgetrennt. Gleichzeitig kann in diesem Schritt auch die Trennung des Konjugats nach der Molekülgröße und somit die Isolierung der niedermolekularen Konjugatfraktion erfolgen.

Die erfindungsgemäßen Konjugate können als Nachweisreagenzien eingesetzt werden. Im Gegenstand der vorliegenden Erfindung ist somit ein Verfahren zum Nachweis eines Analyten in einer Probe durch Kontaktieren der Probe mit einem Analyt-spezifischen Nachweisreagenz, wobei man als Nachweisreagenz eine erfindungsgemäße niedermolekulare Konjugatzusammensetzung aus einem Photoprotein und einem Bindepartner verwendet.

Das erfindungsgemäße Nachweisverfahren kann zur Bestimmung beliebiger Analyten durchgeführt werden, z.B. Antigenen, Haptenen, Antikörpern, Hormonen, Hormonrezeptoren, Metaboliten, Nucleinsäuren oder gesamten Zellen oder Bestandteilen davon.

Die erfindungsgemäße Konjugatzusammensetzung wird dabei als Analyt-spezifisches Nachweisreagenz eingesetzt, deren Bindepartnerkomponente entweder direkt mit dem zu bestimmenden Analyten oder indirekt mit einem weiteren Bindepartner des zu bestimmenden Analyten bindet.

Die Art der Bindung des Nachweisreagenz mit dem Analyten oder dem weiteren Analytbindepartner ist eine Rezeptor-Ligand-Wechselwirkung, d.h. eine hochaffine biospezifische Wechselwirkung, z.B. eine Antigen/Hapten-Antikörper-Wechselwirkung, eine Hormon-Hormonrezeptor-Wechselwirkung, eine Biotin-Streptavidin/Avidin-Wechselwirkung, eine Lectin-Saccharid/Glycoprotein-Wechselwirkung oder eine Wechselwirkung zwischen Nucleinsäuren oder einer Nucleinsäure und einem Nucleinsäureanalogon.

Das erfindungsgemäße Verfahren kann als Einschritt- bzw. als Zwei- oder Mehrschrittverfahren durchgeführt werden, wobei das Inkubationsvolumen pro Reaktionsschritt vorzugsweise maximal 200 µl, besonders bevorzugt 20-100 µl beträgt. Die Inkubationsdauer pro Reaktionsschritt beträgt im Falle immunreaktiver Testformate vorzugsweise maximal 60 min und besonders bevorzugt maximal 45 min und im Falle von Hybridisierungsreaktionen vorzugsweise maximal 120 min und besonders bevorzugt maximal 60 min Das erfindungsgemäße Verfahren kann beispielsweise als immunologisches Nachweisverfahren oder als Nucleinsäure-Hybridisierungsnachweisverfahren durchgeführt werden. Das Testformat kann sowohl kompetitiv als auch nicht-kompetitiv gewählt werden. Weiterhin kann das Verfahren auch als Rücktitrationsverfahren durchgeführt werden.

Besonders vorteilhaft ist das erfindungsgemäße Verfahren für kinetisch kontrollierte Rezeptor-Ligand-Assays, d.h. Testverfahren mit kurzen Inkubationszeiten und daher einer dynamisch veränderlichen Reaktionslage weit ab vom thermodynamisch kontrollierten Gleichgewicht, für miniaturisierte Testverfahren oder für vereinfachte Testformate mit einer verringerten Anzahl von Reaktionsschritten oder einer beliebigen Kombination davon.

Da für solche Verfahren im Vergleich zu klassischen Testverfahren (z.B. > 200-500 µl Probe, Gesamtinkubationszeit mehr als 2 Stunden, oft über Nacht) nur relativ wenige Bindeereignisse eintreten können, ist umso überraschender, daß gerade niedermolekulare Konjugatzusammensetzungen aus Bindepartner und Photoprotein den höhermolekularen Konjugaten, z.B. Netzstrukturen aus jeweils 3-5 Aequorin- und Bindepartnermolekülen überlegen sind. So findet man bei Verwendung der erfindungsgemäßen Konjugate in Nachweisverfahren sehr niedrige chemische Rauschpegel (unspezifische Bindung), nur geringe Verluste an spezifisch gebundenem Signal in Vergleich zu den höhermolekularen Konjugatfraktionen, eine sehr dynamische Reaktionskomplexbildung und somit überlegene Signal-Rausch-Quotienten und einen breiteren, sogar weitgehend linearen Meßbereich, eine bessere Präzision, besonders auf Nullkalibratorniveau und somit eine bessere analytische Sensitivität, z.B. berechnet auf Basis der zweifachen Standardabweichung über bzw. unter dem Mittelwert einer Replikatmessung des Leerwerts, d.h. auf einem 95% Vertrauensniveau.

Weiterhin kann insbesondere bei Verwendung von 1:1 Konjugaten eine höhere Retention der Photoproteinaktivität erreicht werden und somit auch eine bessere Lagerstabilität insbesondere in Lösung.

Die Vorteile des erfindungsgemäßen Verfahrens wurden überprüft und bestätigt anhand einer Reihe verschiedener Testformate, z.B. für eine Einschritt-Simultaninkubation wie auch Mehrschritt-Assays mit sequentieller Inkubation, wobei die Einschritt-Simultaninkubation sowohl im nicht-kompetitiven Testformat (Sandwich-Assay oder Excess Reagent Assay) wie auch im kompetitiven Testformat (Limited Reagent Assay), z.B. für Antigene wie etwa TSH oder HCG oder für Haptene, z.B. Thyroxin, als Analyt erfolgte. Die Tests wurden in Standardtestträgern wie Mikrotiterplatten und in speziell entwickelten miniaturisierten Einwegtestträgern (Dispos) mit nur 40 µl Gesamtreaktionsvolumen durchgeführt.

Als Aequorinbindepartner wurden Immunglobuline, z.B. Maus-IgG Subklasse γ1, aber auch die schwierig zu handhabende Maus-IgG Subklasse γ2b, monoklonale Antikörper, polyklonale Antikörper aus unterschiedlichen Tierspezies (z.B. Schaf, Ziege), intaktes IgG, F(ab')₂-, Fab' und Fab-Fragmente sowie DNA-Oligonucleotide verwendet. Außerdem wurden sowohl Konjugatpräparationen eingesetzt, bei denen die gewünschte niedermolekulare Fraktion aus dem Produktgemisch im Verlauf der Aufreinigung als Teilfraktion isoliert wurde, als auch solche, bei denen eine 1:1 Konjugatstruktur durch Verwendung monofunktionaler Bindereagenzien quasi erzwungen wurde.

Noch ein weiterer Gegenstand der vorliegenen Erfindung ist ein Reagenz zur Durchführung des erfindungsgemäßen Verfahrens, welches eine Konjugatzusammensetzung gemäß vorliegender Erfindung enthält. Die Konjugatzusammensetzung kann als Lösung, Suspension oder Lyophilisat vorliegen. Das Reagenz kann auch als Bestandteil eines Reagenzienkits zum Nachweis eines Analyten in einer Probe vorliegen, der neben dem erfindungsgemäßen Nachweisreagenz auch die für die jeweilige Bestimmung benötigten anderen Testkomponenten enthält.

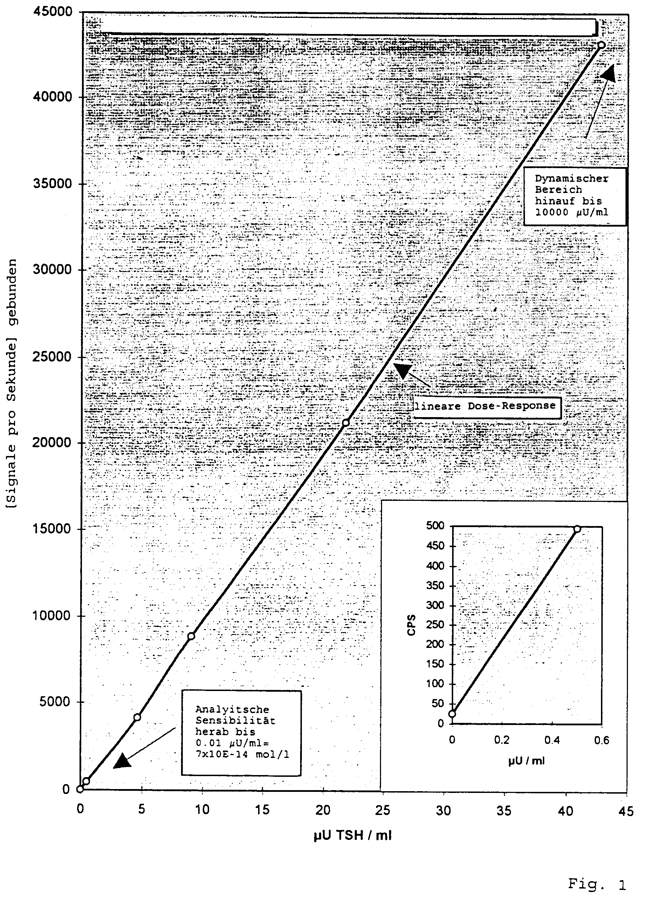

Weiterhin soll die vorliegende Erfindung durch die nachfolgenden Beispiele und Figuren erläutert werden.
- Es zeigen:: Fig. 1 die Dose-Response-Kurve für einen Biolumineszenz-Immunoassay zur Bestimmung von TSH unter Verwendung eines erfindungsgemäßen Aequorin-Konjugats,
Fig. 2 das Elutionsprofil eines Konjugatansatzes von einer Gelfiltrationschromatographiesäule und die Auftrennung in einzelne Pools,
Fig. 3 die Elutionsprofile der einzelnen Konjugatpools von einer HPLC-Säule.

### BEISPIELE

### Materialien

2-Iminothiolan von Pierce, MHS von Boehringer Mannheim, BMH von Pierce, Q-Sepharose FF von Pharmacia, DEAE Bio Gel A von Bio Rad, Sephacryl S 200 HR von Pharmacia, Superose 12 von Pharmacia, Superdex 200 von Pharmacia, Autolumat LB 953 von Berthold.

### Beispiel 1:

### Herstellung von Aequorin-Konjugaten

### 1.1. Derivatisierung von Aequorin mit 2-Iminothiolan

Aequorin in 10 mM Tris, 0.15 M NaCl, pH 8.0, 2 mM EDTA wird in 10 mM Hepes, 0.2 M NaCl, 2 mM EDTA, 2 mM Dithiothreitol (DTT), pH 8.0 dialysiert oder über Sephadex G25 umgepuffert. In diesem Puffer wird die Aktivierung mit 2-Iminothiolan durchgeführt. Die Konzentration des zu aktivierenden Aequorins wird über OD280 unter Berücksichtigung des molaren Extinktionskoeffizienten bestimmt.

Die Aktivierung mit 2-Iminothiolan erfolgt bei einem 15- bis 30-fachen molaren Überschuß an 2-Iminothiolan, bevorzugt bei einem ca. 15-fachen Überschuß. Die Konzentration des Aequorins bei der Aktivierung beträgt 2.4 mg/ml. Die Inkubation erfolgt bei 25°C unter ständigem Rühren im Wasserbad für 30 min. Die Aktivierung wird mit 1 M Lysin-Hydrochlorid in einer Endkonzentration von 10 mM gestoppt und der Ansatz weitere 15 min inkubiert. Der Überschuß an 2-Iminothiolan wird über eine mit 25 mM Hepes, 3 mM EDTA, pH 7.5 äquilibrierte Sephadex G 25 Säule oder eine Dialyse bei +4°C in dem gleichen Puffer abgetrennt. Die Proteinkonzentration wird über OD280 bestimmt.

Unter diesen Bedingungen kann man in das Aequorinmolekül 1 bis 1.5 zusätzliche Sulfhydryl-Gruppen einführen, wobei die Bestimmung der Einbaurate via 4,4'-Dithiodipyridin erfolgen kann. Die spezifische Photoproteinaktivität beträgt ≥ 95% der spezifischen Aktivität des unmodifizierten Aequorins. Das mit 2-Iminothiolan aktivierte Aequorin wird bis zur Konjugationreaktion mit den Maleimidogruppen des Bindepartners im Eisbad oder bei Lagerung über 1 Tag bei -70°C aufbewahrt.

### 1.2. Aktivierung des Bindepartners (Antikörper)

Der Antikörper wird mit einem 1.2- bis 1.7-fachen molaren Überschuß an MHS aktiviert. Die Konzentration des Antikörpers bei der Aktivierung beträgt 20 mg/ml. Lyophilisierter Antikörper wird direkt in 30 mM Natriumphosphatpuffer (NaPP), pH 7.1 gelöst, und ein bereits gelöstes Antiköper (Ak)-Präparat wird in 30 mM NaPP, pH 7.1 umgepuffert. Die Konzentration des zu aktivierenden Antikörpers wird über die OD280 unter Berücksichtigung des entsprechenden molaren Extinktionskoeffizienten bestimmt. Das MHS wird immer frisch in Dimethylsulfoxid (DMSO) gelöst und sofort für die Aktivierung eingesetzt.

Die Inkubation des Reaktionsansatzes erfolgt bei 25°C im Wasserbad unter ständigem Rühren für 60 min. Die Reaktion wird durch Zugabe von 1 M Lysin-Hydrochlorid in einer Endkonzentration von 10 mM gestoppt. Die Inkubation wird dann für weitere 30 min fortgesetzt. Der Überschuß an Aktivierungsreagenz wird über eine mit 25 mM Hepes, 3 mM EDTA, pH 7.5 äquilibrierte Sephadex G 25 Säule oder durch Dialyse in dem gleichen Puffer abgetrennt.

Die Proteinkonzentration wird über OD280 Messung bestimmt. Unter diesen Bedingungen wird ca. 1 Maleimidgruppe in das Molekül des Antikörpers eingeführt. Die Bestimmung der Einbaurate erfolgt über Zugabe von L-Cystein als freie Aminosäure und Rücktitration des unverbrauchten L-Cystein via 4,4'-Dithiodipyridin. Maleimid-aktivierter Antikörper wird bis zur Konjugation mit 2-Iminothiolan-aktiviertem Aequorin im Eisbad aufbewahrt. Für längere Lagerung (> 1 Tag) wird der Maleimidaktivierter Antikörper bei -70°C gelagert.

### 1.3. Konjugation

Das mit 2-Iminothiolan aktivierte Aequorin und der mit Maleimid aktivierte Bindepartner werden in einem molaren Verhältnis 1 : 1 bis 5 : 1, bevorzugt ca. 2,5 : 1 vereint. Die Konzentration des Aequorins im Ansatz beträgt 1 mg/ml. Der Konjugationsansatz wird im Wasserbad unter ständigem Rühren inkubiert.

Der Konjugationsverlauf wird über eine analytische Gelfiltrationschromatographiesäule verfolgt, so daß erst ein Profil der Reaktionskomponenten (Antikörper und Aequorin) in den gleichen Konzentrationen wie in dem Reaktionsansatz aufgenommen wird. Nach dem Reaktionsstart wird dann in 15-min Intervallen das Konjugationsprofil aufgenommen. Die Konjugation wird in der Regel nach 45 - 60 min abgeschlossen. Es entstehen überwiegend niedrigvernetzte Konjugate. Der durch die Aktivierung aggregierende geringe Prozentsatz an Antikörper und Aequorin kann jedoch auch zur Bildung von hochvernetzten Konjugaten führen.

Die freien Maleimidgruppen der Antikörper werden mit 0.2 M Cystein-Hydrochlorid in einer Endkonzentration von 2 mM gestoppt und die Inkubation noch 15 min fortgeführt. Die Restaktivität des Konjugates nach Abstoppen beträgt noch 70 - 90 % der spezifischen Aktivität des unmodifizierten Aequorins.

### 1.4. Aufreinigung

Als erster Schritt wird aus dem Konjugationsansatz, der das Konjugat, freies Aequorin und freien Antikörper enthält, über eine Ionenaustauschchromatographie (IEC) der freie Antikörper abgetrennt. Für die beiden Reaktionspartner werden die Bedingungen für die IEC (Ionenaustauscher, Puffer, Ionenstärke, Gradient) vorher optimiert. Ein Beispiel für einen geeigneten Ionenaustauscher ist Q-Sepharose FF.

Der Konjugationsansatz wird nach Abstoppen der Reaktion in Puffer A (Auftragspuffer oder Bindungspuffer der IEC) umgepuffert und unter den optimierten Bedingungen aufgereinigt. Nach der Auftrennung des Gemisches werden die dem Konjugat zuordenbaren Fraktionen gepoolt und von den Pools die Photoproteinaktivität gemessen. Weiterhin werden Aktivitätsprofile über eine analytische Gelfiltrationschromatographiesäule (wie bei der Konjugation) erstellt. Ein Pool enthält das Konjugat und das freie Aequorin. Die Trennung der beiden Komponenten erfolgt dann über präparative Gelfiltrationschromatographie (GF).

Als Gelfiltrationsmaterial wird Sephacryl S-200 HR, Superose 12, Superdex 200 oder Bio Gel A verwendet. In diesem Schritt wird das freie Aequorin von dem Konjugat und gleichzeitig das Konjugat nach Molekulargröße getrennt. Die Gelfiltrationssäule wird mit 25 mM Hepes, 0.15 M NaCl und 10 mM EGTA, pH 7.4 äquilibriert und die Trennung erfolgt bei +4°C. Eluiert wird mit dem gleichem Puffer und mit einem Fluss von 10% Säulenvolumen/h. Die Fraktionsgröße beträgt 5% des Säulenvolumens.Das Elutionsprofil bei OD280 und die Lichtkurve des Elutionsprofils werden aufgenommen. Die erste nichthomogene Peakformation, in der Regel mehrgipfelig bzw. mit mehreren Schultern, entspricht dem Konjugatgemisch. Die Fraktionen werden so gepoolt, daß man von den Konjugatfraktionen 4 - 6 Pools erhält, in welchen jeweils verschiedene Produktspezies angereichert sind.

Die erste gepoolte Fraktion entspricht einer Anreicherung von hochmolekularen Produkt. Mit fortschreitender Elution verschiebt sich die Verteilung der Konjugatspezies hin zu eher niedermolekularem Produkt - idealerweise 1 : 1 Konjugaten. Die einzelnen Pools werden dann über Amicon YM10 Membran oder Centricon 10 konzentriert. Das Molekulargewicht der Pools wird über eine analytische Gelfiltrationschromatographiesäule bestimmt (unter gleichen Bedingungen wie bei der Konjugation). Die Photoproteinaktivität der Pools wird gemessen und die Proteinkonzentration nach Bradford oder nach der BCA-Methode bestimmt.

Die Pools (in dem Elutionspuffer) werden mit 0.05 % Natriumazid, 0.05% Tween-20 und 0.2 % Rinderserumalbumin konserviert und stabilisiert.

### Beispiel 2:

### Bestimmung von Thyrotropin (TSH)

- Testformat:: 1-Schritt-Simultaninkubation der Reaktanden in Form einer 15 minütigen Inkubation bei Raumtemperatur und mit Schütteln
nicht-kompetitiv, Dose-Response direkt proportional
- Testträger:: entweder weiße Nunc MaxiSorb-Mikrotiterplatten (MTP), dann 75 µl Gesamtreaktionsvolumen, oder miniaturisierte transparente Dispos, dann 40 µl Gesamtreaktionsvolumen; Testträger jeweils mit Streptavidin (SA) beschichtet Probenverdünnung: 1:3, d.h. 25 µl Probe + 50 µl Reagenz (MTP), bzw.
13 µl Probe + 27 µl Reagenz (Dispo)
- Inkubationspuffer:: 100 mM Na-Phosphatpuffer pH 7.4, mit 200 mM Na-Tartrat, 0.5% Synperonic, je 6 mM EDTA und EGTA, je 0.1% Rinderserumalbumin (RSA) und Kaninchen (R)-IgG, 8 µg/l Maus (M)-IgG
- Wandantikörper:: Monoklonaler Anti-TSH-Antikörper (IgG)-Biotinkonjugat, 20 nmol/l
- Kalibratoren:: Matrix: 2% RSA, 0.9% NaCl
Konzentrationsbereich: 0 - ca. 45 µU/ml hTSH
- Konjugat:: a. Monoklonaler Anti-TSH-Mausantikörper V03-(IgG) Aequorin-Konjugat
b. Monoklonaler Anti-TSH-Mausantikörper A8 (IgGγ1) Aequorin-Konjugat
c. Monoklonaler Anti-TSH-Mausantikörper A8 F(ab')₂-Fragment) Aequorin-Konjugat
- Aktivität:: jeweils ca. 2 x 10⁶ cps (Lumo)/Test
- Wascheinheit:: SLT Plate Washer SW812 für MTP, bzw. speziell für die Mini-Dispos entworfener Waschkopf für Dispos
- Triggerlösung:: 10 mM Tris-Puffer pH 7.5 mit 100 mM CaCl₂
- Meßeinheit:: Dynatech ML 3000 Plate Luminometer für MTP im enhanced flash mode, d.h. fixes Auswertungsintegral von 0.2 - 1.2 Sekunden nach Start der Triggerdosierung; bzw. speziell für die transparenten Mini-Dispos entworfener Luminometer (Lumo) mit Triggerzugabe von oben und Messung von unten, mit fixem Auswertungsintegral von 0.2 - 1.2 Sekunden nach Start der Triggerdosierung; Triggervolumen 100 µl bei ML 3000 und 40 µl bei Lumo

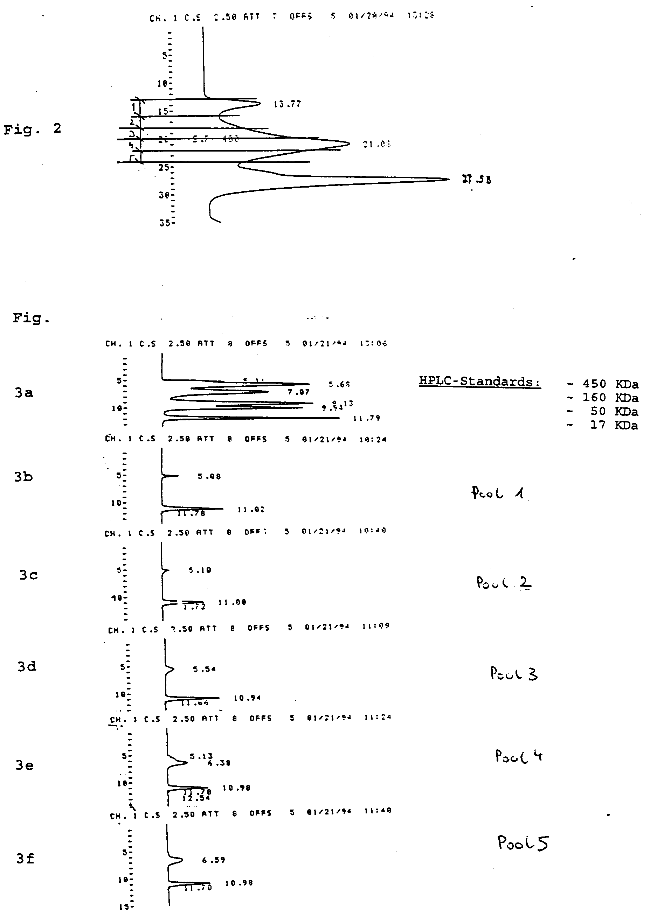

Die Konjugate (a), (b) und (c) werden gemäß der in Beispiel 1 beschriebenen Prozedur hergestellt. Bei der Gelfiltration werden die Fraktionen in mehrere Pools unterteilt. Bei Konjugat (a) enthalten die Pools 1 und 2 ausschließlich und Pool 3 überwiegend hochvernetztes Konjugat, während die Pools 4 und 5 die gewünschten niedermolekularen Konjugatfraktionen enthalten (vgl. auch Fig. 2 und 3). Bei Konjugat (b) enthält Pool 1 hochvernetztes Konjugat, Pool 2 eine Mischung von hoch- und niedrigvernetztem Konjugat, während in Pool 3 das gewünschte niedrigmolekulare Konjugat vorliegt. Pool 4 enthält bereits große Anteile an vernetzem Aequorin und anderen Nebenprodukten und liefert daher keine so guten Resultate. Bei Konjugat (c) enthalten die Pools 1 und 2 hochvernetzte Konjugate und die Pools 3 bis 5 niedrigvernetzte Konjugate.

Die Ergebnisse sind in den Tabellen 1 bis 3 dargestellt.

Aus den Tabellen ist ersichtlich, daß unabhängig vom verwendeten Antikörper (MAK V03 bzw. MAK A8) und der Antikörper-Präparation (MAK A8 - intaktes IgG bzw. F(ab')₂) im Gang von Pool 1 (= praktisch ausschließlich hochmolekulares Material) zu Pool 4 bzw. 5 (= praktisch ausschließlich niedermolekulares Material) die unspezifische Bindung signifikant abnimmt, während die Absolutdifferenz an spezifischer Bindung nur moderat geringer wird und das Signal:Rausch-Verhältnis (Kurvensteilheit) somit kräftig zunimmt.
Zur Verdeutlichung bei Konjugat (c):
- Pool 1:: Nullwertpräzision = 30.1%
Steilheit B/A = 3.07
untere Nachweisgrenze = 0.15 µU/ml
- Pool 4:: Nullwertpräzision = 31.25%
Steilheit B/A = 8.75
untere Nachweisgrenze = 0.04 µU/ml

Gute Kurvensteilheit, in Verbindung mit guter Nullwertpräzision, ist die Basis für gute analytische Sensitivität (Eichkurven-Empfindlichkeit). Je geringer die unspezifische Bindung, desto besser die Steilheit der Kurve bei mindestens gleichbleibender Nullwertpräzision, und deshalb sinkt die untere Nachweisgrenze meistens in Richtung niedermolekulare Konjugatpools immer weiter ab, d.h. die Sensitivität steigt beträchtlich.

Dieser Zusammenhang wird auch besonders deutlich beim Übertrag des TSH-Assays vom Dispo-System zum etablierten Mikrotiterplattensystem (d.h. u.a. vorteilhaftes Arbeiten mit Mehrkanalpipetten, 8- bzw. 16-fach-Nadelwaschköpfen, etc.), wie in Tab. 1 anhand von Konjugat (a) Pool 3 verdeutlicht.
- Dispo-System:: Nullwertpräzision = 38.8%
Steilheit B/A = ca. 4.9
untere Nachweisgrenze = 0.10 µU/ml
- MTP-System:: Nullwertpräzision = 20.2%
Steilheit B/A = 19.3
untere Nachweisgrenze = 0.01 µU/ml (7·10⁻¹⁴ mol/l)

Anhand der S/N-Daten und der Dose-Response-Korrelation wird außerdem die hervorragende Linearität der Eichkurven unterstrichen. Die lineare Meßbereichsspanne reicht von 0.01 - 10000 µU/ml (Fig. 1).

Zu beachten ist, daß der letzte Pool am niedermolekularen Ende der Konjugatverteilungskurve in manchen Fällen aggregiertes Aequorin und andere Nebenprodukte enthalten kann, so daß z.T. der zweitletzte Pool die besten Resultate liefert (vgl. z.B. Tab. 2, Pool 3 und 4.

Die aktivste Teilfraktion ist durch den überwiegenden Gehalt an Reaktionsprodukt mit einem Molekulargewicht von ca. 170 kD im Falle von IgG als Bindepartner charakterisiert, wie ein Abgleich an einer kalibrierten Gelfiltrationschromatographiesäule zeigt. In Fig. 2 ist das Elutionsprofil des Konjugatansatzes (a) von der Gelfiltrationschromatographiesäule (Superose 12) anhand einer UV-Messung (OD280) sowie die Auftrennung in die einzelnen Pools 1, 2, 3, 4 und 5 dargestellt. Fig. 3 zeigt die Elutionsprofile der einzelnen Pools von einer HPLC-Säule (TSK3000). In Fig. 3a sind verschiedene HPLC-Standards mit Molekulargewichten von 17 bis 450 kD dargestellt. Der Referenzstandard für 150 - 170 kD hat eine Retentionszeit von ca. 7 min. In Fig. 3b bis d sind die überwiegend hochmolekularen Konjugate (Retentionszeit 5 bis 6 min) enthaltenden Pools 1 bis 3 gezeigt. Die bei einer Retentionszeit von ca. 11-12 min auftretenden Peaks stammen vom Puffer. Aus Fig. 3c ist ersichtlich, daß Pool 4 bereits einen relativ hohen Anteil an erwünschtem niedermolekularen Konjugat (Retentionszeit 6,38) mit einer Schulter bei 5,13 min, was auf geringe hochmolekulare Verunreinigungen hindeutet, aufweist. Der in Fig. 3f gezeigte Pool 5 enthält ebenfalls hohe Anteile an erwünschtem niedermolekularen Konjugat (Retentionszeit 6,59 min) ohne meßbare Anteile an hochmolekularem Produkt, aber eventuell mit Anteilen an aggregiertem Aequorin, so daß die bei konstantem Einsatz von 2 x 10⁶ cps/Test die Testdynamik (siehe Tab. 1) bereits wieder leicht sinkt.

Das in den Elutionsprofilen gemäß Fig. 3 ersichtliche niedermolekulare Konjugat entspricht eindeutig aufgrund seiner Mobilität verglichen mit HPLC-Standards einem 1:1-Kopplungsprodukt aus einem Molekül IgG (ca. 150 kD) und einem Molekül Aequorin (ca. 22 kD) mit Immunreaktivität und Photoproteinaktivität. Folglich ist die niedermolekulare Konjugatfraktion der Schlüssel zu einer signifikanten Leistungssteigerung des Immunoassays.

### Beispiel 3:

### Bestimmung von Humanchoriongonadotropin (HCG)

- Testformat:: 1-Schritt-Simultaninkubation der Reaktanden in Form einer 15 minütigen Inkubation bei Raumtemperatur und mit Schütteln nicht-kompetitiv, Dose-Response direkt proportional
- Testträger:: weiße Nunc MaxiSorb-Mikrotiterplatten, SA-beschichtet,
60 µl Gesamtreaktionsvolumen
- Probenverdünnung:: 1:6, d.h. 10 µl Probe + 50 µl Reagenz
- Inkubationspuffer:: 40 mM Na-Phosphatpuffer pH 7.4, mit 300 mM NaCl, 0.2% RSA, 0.1% R-IgG, 1% Synperonic sowie je 6 mM EDTA und EGTA
- Wandantikörper:: Monoklonaler Anti-HCG-Mausantikörper (IgG)-Biotinkonjugat, 20 nmol/l
- Kalibratoren:: Matrix: 2% RSA / 0,015% Lipoprotein
Konzentrationsbereich: 0 - ca. 19000 U/ml HCG
- Konjugat:: Monoklonaler Anti-HCG-Mausantikörper (IgGγ1)-Aequorinkonjugat
- Aktivität:: ca. 2x 10⁶ cps (Lumo)/Test
- Wascheinheit:: SLT Plate Washer SW812
- Triggerlösung:: 10 mM Tris-Puffer pH 7.5 mit 100 mM CaCl₂
- Meßeinheit:: Dynatech ML 3000 Plate Luminometer für MTP im integrated flash mode, d.h. via Peakerkennung und 0.1 sec vor-Peak- und 2 sec nach-Peak-Integration

Die Herstellung und Aufreinigung des Konjugats erfolgt wie in Beispiel 1 beschrieben. Bei der Aufreinigung werden insgesamt 4 Pools erhalten. Pool 1 enthält hochvernetztes Konjugat. Pool 2 enthält eine Mischung von niedrig- und hochvernetzten Konjugaten. Die Pools 3 und 4 enthalten niedrig vernetzte Konjugate.

Die Ergebnisse sind in Tabelle 4 dargestellt. Im Prinzip werden die Ergebnisse von Beispiel 2 bestätigt.

Mit der Anreicherung an niedermolekularem Anteil an Konjugationsprodukt (Pool 3-4) wird das Maximum an Differenzierung (S/N) und Sensitivität erreicht. Pool 5 enthält bereits nennenswerte Anteile an Nicht-Konjugat-Bestandteilen.

Im besonderen zu beachten ist hier die ganz außergewöhnlich breite Meßbereichsspanne auf Konzentrationsebene (ca. 1-20000 mU/ml, gegenüber ca. 1-500 mU/ml beim Stand der Technik, z. B. beim Enzymum®-Test HCG) und dies in einem Einschritt-Assay. Dadurch wird die enorme Dynamik dieser Konjugate illustriert.

### Beispiel 4:

### Bestimmung eines Cytokeratinfragments (CK 19)

- Testformat:: 1-Schritt-Simultaninkubation der Reaktanden in Form einer 15 minütigen Inkubation bei Raumtemperatur und mit Schütteln
nicht-kompetitiv, Dose-Response direkt proportional
- Testträger:: weiße Nunc MaxiSorb-Mikrotiterplatten, SA-beschichtet,
60 µl Gesamtreaktionsvolumen
- Probenverdünnung:: 1: 6, d.h. 10 µl Probe + 50 µl Reagenz
- Inkubationspuffer:: 50 mM Na-Phosphatpuffer pH 7,4, mit 150 mM NaCl, 0.6% Synperonic, je 10 mM EDTA und EGTA, 1% PEG 40000, 1% RSA, 0.2% R-IgG, 20 mg/l M-IgG
- Wandantikörper:: Monoklonaler Anti-CK19-Mausantikörper (Fab-Fragment)-Biotinkonjugat, 1 µg/ml
- Kalibratoren:: Matrix: 6% RSA / 0.9% NaCl
Konzentrationsbereich: 0 - 44 ng/ml CK19
- Konjugat:: Monoklonaler Anti-CK19-Mausantikörper (IgGγ1)-Aequorin-Konjugat
- Aktivität:: ca. 2 x 10⁶ cps (Lumo)/Test
- Wascheinheit:: SLT Plate Washer SW812
- Triggerlösung:: 10 mM Tris-Puffer pH 7.5 mit 100 mM CaCl₂
- Meßeinheit:: Dynatech ML 3000 Plate Luminometer für MTP im enhanced flash mode, d.h. fixes Auswertungsintegral von 0.2 - 1.2 Sekunden nach Start der Triggerdosierung

Die Herstellung des Antikörper-Aequorin-Konjugats erfolgt wie in Beispiel 1 beschrieben. Nach Gelfiltration werden insgesamt 6 Pools erhalten. Die Pools 1 und 2 enthalten hochvernetzte Konjugate. Die Pools 3 und 4 enthalten die höchste Anreicherung an niedermolekularen Konjugaten. Der Pool 5 und insbesondere der Pool 6 enthalten vermutlich freies IgG.

Die Ergebnisse dieses Versuchs sind in Tabelle 5 zusammengefaßt. Im Prinzip werden die in Beispiel 2 erhaltenen Ergebnisse bestätigt.

Mit der Anreicherung des niedermolekularen Anteils an Konjugationsprodukt (Pool 4-5) wird das Maximum an Differenzierung (S/N), sowohl im unteren als auch oberen Konzentrationsbereich, und an Sensitivität erreicht. Pool 6 enthält bereits nennenswerte Anteile an Nicht-Konjugat-Bestandteilen, wie z.B. Aequorin-Aggregate, was in erhöhter unspezifische Bindung sowie reduzierten spezifischen Bindungsniveaus resultiert.

### Beispiel 5:

### Bestimmung von Gesamt-IgE

- Testformat:: 3-Schritt-Assay mit sequentieller Inkubation von biotinyliertem Wandantikörper, Probe, und Konjugat für jeweils 20 Minuten bei Raumtemperatur und unter Schütteln.
nicht-kompetitiv, Dose-Response direkt proportional
- Testträger:: weiße Nunc MaxiSorb-Mikrotiterplatten, SA-beschichtet,
50 µl Gesamtreaktionsvolumen pro Testschritt
- Probenverdünnung:: 1:2, d.h. 25 µl Probe + 25 µl Inkubationspuffer
- Inkubationspuffer:: 100 mM Tris-Puffer pH 7.6, mit 400 mM KCl, 0.2% RSA, 0.5% Synperonic
- Wandantikörper:: Monoklonaler Anti-Human-IgE-Mausantikörper (IgG)-Biotinkonjugat, 0.1 µg/ml
- Kalibratoren:: Matrix: Pferdeserum
Konzentrationsbereich: 0 - ca. 450 U/ml
- Konjugat:: Monoklonaler Anti-Human-IgE-Mausantikörper (IgGγ2b)-Aequorinkonjugat
- Aktivität:: ca. 1 x 10⁶ cps (Lumo)/Test
- Wascheinheit:: SLT Plate Washer SW812
- Triggerlösung:: 10 mM Tris-Puffer pH 7.5 mit 100 mM CaCl₂
- Meßeinheit:: Dynatech ML 3000 Plate Luminometer für MTP im enhanced flash mode, d.h. fixes Auswertungsintegral von 0.2 - 1.2 Sekunden nach Start der Triggerdosierung

Die Herstellung des Konjugats erfolgt wie in Beispiel 1 beschrieben. Bei der Gelfiltration werden die Konjugatfraktionen in 5 Pools aufgetrennt. Die Pools 1 und 2 enthalten hochvernetzte Konjugate, die Pools 3 und 4 niedrigvernetzte Konjugate. Pool 5 enthält vermutlich Spuren an IgG.

Die Ergebnisse sind in Tabelle 6 dargestellt.

Gerade im Vergleich von Pool 1 zu Pool 4 (= niedermolekulare Fraktion) werden auch im sequentiellen Mehrschrittformat eine drastische Verminderung der unspezifischen Bindung, praktisch keine Verluste an spezifischer Bindung, und somit eine enorme Zunahme der Eichkurvensteilheit deutlich, was sich in einer kontinuierlichen Verbesserung der Sensitivität niederschlägt.

Pool 5 enthält bereits nennenswerte Anteile an nicht reaktiven Bestandteilen, was zu reduzierter Dynamik und geringerer spezifischer Bindung führt.

### Beispiel 6:

### Bestimmung von Troponin T (TN-T)

- Testformat:: 1-Schritt-Simultaninkubation der Reaktanden in Form einer 15 minütigen Inkubation bei Raumtemperatur und mit Schütteln
nicht-kompetitiv, Dose-Response direkt proportional
- Testträger:: weiße Nunc MaxiSorb-Mikrotiterplatten, SA-beschichtet,
60 µl Gesamtreaktionsvolumen
- Probenverdünnung:: 1:6, d.h. 10 µl Probe + 50 µl Reagenz
- Inkubationspuffer:: 50 mM Na-Phosphat / 10 mM Na-Citrat-Puffer pH 6.0, mit je 6 mM EDTA und EGTA, 0.1% RSA, 0.5% Synperonic
- Wandantikörper:: Monoklonaler Anti-TN-T-Mausantikörper (IgG)-Biotinkonjugat, 7 µg/ml
- Kalibratoren:: Matrix: 6% RSA, 0.9% NaCl
Konzentrationsbereich: 0 - ca. 16.5 ng/ml
- Konjugat:: Monoklonaler Anti-TN-T-Mausantikörper (IgGγ1)-Aequorinkonjugat
- Aktivität:: ca. 2 x 10⁶ cps (Lumo)/Test
- Wascheinheit:: SLT Plate Washer SW812
- Triggerlösung:: 10 mM Tris-Puffer pH 7.5 mit 100 mM CaCl₂
- Meßeinheit:: Dynatech ML 3000 Plate Luminometer für MTP im enhanced flash mode, d.h. fixes Auswertungsintegral von 0.2 - 1.2 Sekunden nach Start der Triggerdosierung

Die Herstellung des Konjugats erfolgt wie in Beispiel 1 beschrieben. Nach Gelfiltration werden 5 Fraktionen erhalten. Die Pools 1 und 2 enthalten überwiegend hochvernetzte Konjugat, die Pools 3 bis 5 enthalten niedrigvernetzte Konjugate.

Die Ergebnisse des Versuchs sind in Tabelle 7 dargestellt.

Die zuvor in Beispiel 2 gemachten Bemerkungen treffen hier im Gang von Pool 1 (= hochmolekulares Material stark angereichert) zu Pool 5 (= praktisch ausschließlich niedermolekulare Konjugatspezies) in idealer Weise zu.

### Beispiel 7:

### Gesamt-Thyroxin-Assay

Der in diesem Test verwendete Anti-Creatinkinase Subtyp MM (CK-MM)-Antikörper hat mit den übrigen Testreagenzien eine derart geringe spezifische Affinität, daß er als quasi inertes Trägerprotein dient.
- Testformat:: 1-Schritt-Simultaninkubation der Reaktanden in Form einer 15 minütigen Inkubation bei Raumtemperatur und mit Schütteln
kompetitiver Assay im labelled analog-Format mit trägervermittelter Tracer-Technologie; Dose-Response invers proportional
- Testträger:: weiße Nunc MaxiSorb-Mikrotiterplatten, SA-beschichtet,
54 µl Gesamtreaktionsvolumen
- Probenverdünnung:: 1:25, d.h. 2 µl Probe + 2 µl Tracer (Konjugat aus Trägerprotein, Thyroxin-Derivat, und Aequorin) + 50 µl Reagenz
- Inkubationspuffer:: 120 mM Na-Barbitalpuffer pH 8.7, mit 0.04% ANS, 10 mM EGTA, 0.2% RSA
- Wandantikörper:: Polyklonaler Anti-Thyroxin-Schafantikörper-Biotinkonjugat, 0.2 µg/ml
- Kalibratoren:: Matrix: 6 % RSA, 0.9% NaCl, 10 mg/l Thyroxin-Bindendes Globulin (TBG) II
Konzentrationsbereich: 0 - ca. 25 µg/dl
- Konjugat: Monoklonaler Anti-CK-MM-Mausantikörper
- (=Tracer):: (IgGγ1); konjugiert mit Thyroxin-(O-methyl) -N-hemisuccinyl-NHS-ester und nach MH-Aktivierung mit SH-aktiviertem Aequorin, auf 11.4 nmol/l Thyroxin-Reaktivitätsäquivalente eingestellt
- Wascheinheit:: SLT Columbus Plate Washer
- Triggerlösung:: 10 mM Tris-Puffer pH 7.5 mit 100 mM CaCl₂
- Meßeinheit:: Dynatech ML 3000 Plate Luminometer für MTP im integrated flash mode, d.h. via Peakerkennung und 0.1 sec vor-Peak- und 2 sec nach-Peak-Integration

Für diesen Test wird das Aequorin über ein Trägerprotein (unspezifisches Maus-IgG) an das Hapten T4 gebunden. Hierzu wird ein T4 (O-Me) N-Succinimidyl-Derivat zunächst an Aminogruppen des IgG gekoppelt. Danach wird wie in Beispiel 1 beschrieben ein Aequorinkonjugat hergestellt. Bei der Gelfiltrationschromatographie werden insgesamt 5 Fraktionen erhalten. Die Pools 1 und 2 enthalten hochvernetzte Konjugate. Pool 3 enthält die höchste Anreicherung an niedermolekularem Konjugat. Die Pools 4 und 5 enthalten bereits eine größere Menge an freiem IgG.

Die Ergebnisse des Versuchs sind in Tabelle 8 dargestellt.

Die beste Differenzierung wird mit Pool 3 erreicht. Dieser ist charakterisiert durch geringe Anteile an höhermolekularen Spezies, keine Beimischung von Nicht-Konjugat-Anteilen, und sehr starker Anreicherung von niedermolekularem Konjugationsprodukt. Die Pools 4 und 5 weisen dagegen nennenswerte Kontamination mit Nicht-Konjugat-Bestandteilen (z.B. nicht gekoppeltes IgG) auf, was sich bemerkbar macht an einer gedämpften Eichkurvendynamik.

### Beispiel 8:

Das in diesem Test verwendete Biotin/DIG-Peptid wird wie durch Kamber und Hiskey, in: Gross E. & Meienhofer J., The Peptides, Academic Press, New York, 1981, Seiten 145-147 beschrieben, hergestellt. Nach N-terminaler Derivatisierung mit Fmoc-ε-Aminocapronsäure erfolgt die Biotinylierung des Peptids via Umsetzung mit D-Biotin am Trägerharz. Die Digoxigenylierung erfolgt in Lösung nach Abspaltung vom Trägerharz durch Umsetzung mit Digoxigenin-3-O-methylcarbonyl-N-hydroxysuccinimidylester mit dem C-terminal Triethylenglykolacetyl-Lysin-funktionalisierten Peptid zum Endprodukt D-Biotinoyl-ε-amidocaproyl-Ser-Gln-Asn-Tyr-Pro-Ile-Val-amidotriethylenglykolactetyl-ε-digoxigenin-3-O-methylcarbonyl-Lys-OH.

### Digoxigenin (DIG) : Anti-DIG-Bindetest

- Testformat:: 1-Schritt-Simultaninkubation der Reaktanden in Form einer 15 minütigen Inkubation bei Raumtemperatur und mit Schütteln
direkter Bindetest mit doppelt funktionalisiertem Biotin/DIG-Peptid und Anti-DIG-Antikörper-Aequorinkonjugat auf SA-Festphase, Dose-Response direkt proportional
- Testträger:: miniaturisierte transparente Dispos, 40 µl Gesamtreaktionsvolumen; SA-beschichtet
- Probenverdünnung:: 1:3, d.h. 13 µl Probe (= BIO/DIG-Peptid) + 27 µl Reagenz
- Inkubationspuffer:: 25 mM HEPES pH 7.0, mit 150 mM NaCl, 0.2% RSA, 0.15% RSA-c, 10 mM EGTA, 0.05% Tween-20
- Wandantikörper:: ---
- Kalibratoren:: Matrix: Peptid-Konzentrationsreihe in HEPES-Puffer
Konzentrationsbereich: 0 - 1000 pmol/l Biotin/DIG-Heptapeptid
- Konjugat:: Polyklonaler Anti-DIG-Schafantikörper (Fab)-Aequorinkonjugat
- Aktivität:: ca. 2 x 10⁶ cps (Lumo)/Test
- Wascheinheit:: speziell für die Mini-Dispos entworfener Waschkopf für Dispos
- Triggerlösung:: 10 mM Tris-Puffer pH 7.5 mit 100 mM CaCl₂
- Meßeinheit:: speziell für die transparenten Mini-Dispos entworfener Luminometer (Lumo) mit Triggerzugabe von oben und Messung von unten, mit fixem Auswertungsintegral von 0.2 - 1.2 Sekunden nach Start der Triggerdosierung; Triggervolumen 40 µl bei Lumo

Die Herstellung des Konjugats erfolgt wie in Beispiel 1 beschrieben. Bei der Gelfiltration werden 6 Fraktionen erhalten. Die Pools 1 bis 3 sind hochvernetzte Konjugate. Die Pools 4 bis 6 enthalten niedermolekulare Konjugatspezies in steigendem Anreicherungsgrad.

Die Ergebnisse des Versuchs sind in Tabelle 9 zusammengefaßt.

Je stärker die Anreicherung an niedermolekularem Konjugationsprodukt (=> Pool 5-6), desto niedriger die unspezifische Bindung ohne Verlust an spezifischer Bindung, desto höher das Signal-Rausch-Verhältnis, desto besser auch die Dose-Response-Korrelation, und desto höher die Sensitivität.

Für alle bisherigen Beispiele wurden Konjugatpräparationen eingesetzt, bei denen die angestrebte [1:1]-Fraktion aus dem Produktgemisch im Verlauf der Aufreinigung als Teilfraktion herausgeschnitten wurde. Die beiden folgenden Beispiele sind solche, bei denen die eine [1:1]-Konjugatstruktur per Syntheseansatz quasi erzwungen werden kann.

### Beispiel 9:

Das für diesen Test verwendete DNP-Oligonucleotid ist ein 5'-Biotin-modifiziertes 48 Basen langes Oligonucleotid, das nach dem Standard-Phosphoramiditverfahren auf einem Gene Assembler (Pharmacia) als Homo-dT-Oligomer synthetisiert wird. Nur an Position 47 wird das Oligo via Einbau von 1-Dimethoxytrityl-3-O-(2,4-dinitrophenyl)-3-aminopropyl)-triethylenglykolglyceryl-2-O-(2-cyanoethyl)-(N,N-diisopropyl) Phosphoramidit (Clontech, Kat. Nr. 10-1985 xx) funktionalisiert.

### Dinitrophenol (DNP): Anti-DNP-Bindetest

- Testformat:: 1-Schritt-Simultaninkubation der Reaktanden in Form einer 15 minütigen Inkubation bei Raumtemperatur und mit Schütteln
direkter Bindetest mit doppelt funktionalisiertem Biotin/DNP-48mer-Oligonucleotid und Anti-DNP-Aequorinkonjugat auf SA-Festphase, Dose-Response direkt proportional
- Testträger:: weiße Nunc MaxiSorb-Mikrotiterplatten, SA-beschichtet,
75 µl Gesamtreaktionsvolumen
- Probenverdünnung:: 1:3, d.h. 25 µl Probe (= BIO/DNP-Oligonucleotid) + 50 µl Reagenz
- Inkubationspuffer:: 25 mM HEPES pH 7.0, mit 150 mM NaCl, 0.2% RSA, 0.15% RSA-c, 10 mM EGTA, 0.05% Tween-20
- Wandantikörper:: ---
- Kalibratoren:: Matrix: Oligonucleotid-Konzentrationsreihe in HEPES-Puffer
Konzentrationsbereich: 0 - 1000 pmol/l Biotin/DNP-Oligonucleotid
- Konjugat:: Polyklonaler Anti-DNP-Ziegenantikörper (Fab'-Fragment) Aequorinkonjugat
- Aktivität:: ca. 1-3 x 10⁶ cps (Lumo)/Test, abhängig von der verfügbaren Stammlösungskonzentration
- Wascheinheit:: SLT Plate Washer SW812
- Triggerlösung:: 10 mM Tris-Puffer pH 7.5 mit 100 mM CaCl₂
- Meßeinheit:: Dynatech ML 3000 Plate Luminometer für MTP im enhanced flash mode, d.h. fixes Auswertungsintegral von 0.2 - 1.2 Sekunden nach Start der Triggerdosierung

Zur Herstellung des Aequorinkonjugats wird das F(ab')₂-Fragment mit Cysteamin reduziert. In das aus der Reduktion resultierende Fab' wird durch die Aktivierung mit einem homobifunktionellen Linker (Bis-maleimidomethylether) eine Maleimidgruppe über die einzige Sulfhydrylgruppe des Ziegen-Fab'-Fragments eingeführt. Somit wird durch den Einbau einer einzigen Maleimidgruppe in das Fab'-Molekül die Bildung von Konjugaten in einer Stöchiometrie 1:1 erzwungen.

Bei diesem Antikörper ist eine Aufreinigung über Ionenaustauschchromatographie aufgrund mangelnder Löslichkeit bei Salzgehalten unter 200 mM Na⁺ nicht möglich. Die Aufreinigung über Gelfiltrationschromatographie ergibt 3 Fraktionen. Pool 1 enthält hochvernetztes Konjugat. Pool 2 enthält überwiegend den niedermolekularen Anteil des Konjugats. Pool 3 enthält vermutlich nur freies Aequorin. Die Trennung ist aufgrund der "Klebrigkeit" des Antikörperpräparats nicht vollständig.

Die Ergebnisse des Versuchs sind in Tabelle 10 dargestellt.

Auch hier wird klar, daß Pool 2, der den niedermolekularen Anteil an Konjugat enthielt (Pool 3 enthielt hingegen fast überhaupt kein aktives Konjugat), das beste Resultat liefert.

### Beispiel 10:

### DNA-Hybridisierungsassay

### 1. Herstellung von DNA-Aeqnorin-Konjugaten

Die Synthese der zur natürlichen Chlamydia trachomatis-Sequenz komplementären Oligonucleotide erfolgt mit Standard-Phosphoramidit-Chemie auf einem Gene Assembler Plus der Firma Pharmacia nach den Standardprotokollen der Bedienungsanleitung und den Angaben der Phosphoramidithersteller.

Als Nucleotid-Bausteine werden die Standardphosphoramidite 5'-Dimethoxytrityl-N-benzoyl-2'-deoxy-adenosin-3'-[(2-cyanoethyl)-(N,N-diisopropyl)]-phosphoramidit, 5'-Dimethoxytrityl-N-benzoyl-2'-desoxy-cytidin-3'[(2-cyanoethyl)-(N,N-diisopropyl)]phosphoramidit,5'-Dimethoxytrityl-N-isobutyryl-2'-desoxyguanosin-3'-[(2-cyanoethyl)-(N,N-diisopropyl)]-phosphoramidit und 5'Dimethoxytrityl-2'-desoxy-thymidin-[(2-cyanoethyl)-(N,N-diisopropyl)]-phosphoramidit ( Firma ABI) verwendet. Nach 5 Stunden bei 55°C in einer 2%igen Ammoniaklösung werden Oligonucleotide vom Träger und gleichzeitig die β-Cyanoethyl-Gruppen sowie die basenlabilen Aminoschutzgruppen abgespalten. Die Rohprodukte werden durch HPLC (Lichrosorb RP 18, 8 X 250 mm, von Chromatographie Service) über einen 0,1 M Triethylammoniumacetat / Acetonitril-Gradienten gereinigt und mittels Dialyse entsalzt.

Von der Basis-Sequenz 5'-CAT AGC ACT ATA GAA CTC TG-3' ausgehend werden 3 Aminoalkyl-Funktionalisierungen vorgenommen:
I.5'-terminale Derivatisierung durch Anhängen von [N-Trifluoracetamido-(3-oxa)pentyl-N,N-diisopropyl-methyl]-phosphoramidit als letzter Syntheseschritt. Nach Abspaltung des Trifluoracetat-Restes durch Baseneinwirkung entsteht ein Oligonucleotid mit einer 5'-terminalen primären Aminogruppe an einem Alkylrest. Hier kann dann die Umsetzung mit einem Maleinimidreagenz wie z. B. MHS oder SMCC erfolgen, so daß ein 5'-MH-aktiviertes Oligonucleotid entsteht, welches dann mit niedrig SH-aktiviertem Aequorin zur Reaktion gebracht werden kann. Daraus resultiert dann ein Konjugat mit einem stöchiometrischen Verhältnis der Komponenten von primär 1:1, wobei die Kopplung auf seiten des Oligonucleotids positional und numerisch determiniert ist.
II. Derivatisierung innerhalb der Sequenz durch Einbau von N-(9-Fluorenylmethoxycarbonyl)-1-dimethoxytrityl-3-(β-cyanoethoxy-N,N-diisopropylaminophosphinyl)-4-amido-(3,6-dioxa-1,8-diamino-octan)-3,3-dimethylbutyrat an Position 11, d.h. Einführung einer Basenleerstelle zugunsten einer wiederum primären Aminogruppe an einem Alkylrest in Form eines 8-Amino-3,6-dioxa-octan-1-amido-3,3-dimethylbutyryl-2,4-phosphoryl-di-esters. Damit ist wiederum der Anschluß an die MH-SH-Kopplungschemie gegeben analog I.
III. Derviatisierung innerhalb der Sequenz durch Einbau einer Aminoalkyl-funktionalisierten Nucleobase, hier [5-(N-(Amino-hexyl)-3-acrylimido)-2'-deoxyuridyl-N,N-diisopropyl-β-cyanoethyl]-phosphoramidit. An der endständigen primären Aminoalkylgruppe kann wiederum MH-Aktivierung und weiter die Kopplung an niedrig SH-aktiviertes Aequorin erfolgen. Resultat ist wie bei I und II ein auf Oligonucleotid-Seite positional und numerisch prädetermiertes Konjugat, welches aufgrund der sehr niedrig gewählten SH-Aktivierung des Aequorins primär 1:1-Reaktionsprodukt enthält.

Die MH-Aktivierung der Aminoalkyl-funktionalisierten Oligonucleotide und die nachfolgende Kopplung an SH-aktiviertes Aequorin wird in folgender Weise durchgeführt:

Amino-modifiziertes Oligonucleotid wird in einer Ausgangskonzentration von ca. 100 µmol/l (ca. 20 nmol im Reaktionsansatz) in 0,1 M NaHCO₃-Puffer pH 8.5 MH-derivatisiert via Umsetzung mit SMCC, als Konzentrat gelöst in Dimethylformamid (DMF), zur Unterdrückung von Hydrolyse vor Reaktionsstart. Davon wird ein kleines Volumen zugegeben, so daß sich ein 10-facher molarer Überschuß zum Oligonucleotid einstellt und der Volumenanteil DMF unter 5% bleibt. Eine Inkubation von 4 Stunden bei Raumtemperatur unter Schütteln sorgt für eine quasi quantitative Umsetzung. Überschüssiges Ausgangsmaterial wird über NAP 10-Säulen der Fa. Pharmacia abgetrennt.

Aequorin wird, wie im Beispiel 1 beschrieben, niedrig SH-aktiviert (ca. 1.5 zusätzliche SH-Gruppen pro Molekül Aequorin).

Schließlich werden beide aktivierte Komponenten in einer Stöchiometrie von Aequorin-SH: Oligonucleotid-MH = 2.5 : 1 unter Bildung einer stabilen Thioetherbrücke gekoppelt. Die Kopplungsbedingungen (Puffer, Stoppen) sind wie in Beispiel 1 beschrieben.

Dann erfolgt eine 1-Schritt-Aufreinigung über Hi-Trap-Q-Säule (Pharmacia) und Gradienten-Elution (Auftragepuffer = 10 mM Tris, 2 mM EDTA, pH 8.0; Elution via stufenweisem Waschen mit Auftragepuffer, Auftragepuffer + 0,25 M NaCl, 0.5 M bzw. 1.0 M NaCl). Das Konjugat eluiert als ein scharfer Peak mit 0,5 M NaCl und 19-21 Retentionsminuten, klar getrennt von freiem Aequorin, welches mit 0,25 M NaCl und 10-17 Retentionsminuten von der Säule kommt.

Zur funktionellen Überprüfung wird das Konjugat auf ca. 1-2 x 10⁶ RLU/Test in Testpuffer (s.U.) verdünnt.

### 2. Testdurchführung

Die Nucleotidsequenz der biotinylierten Fangsonde ist wie folgt:
5'-Biotin-CCC CCC CCC CCA GAG TTC TAT AGT GCT ATG-3'
- Testformat:: 1-Schritt-Simultaninkubation der Reaktanden in Form einer 60 minütigen Inkubation bei 37°C und mit Schütteln (180 rpm)
direkter Bindetest mit biotinylierter Fangsonde (30mer-Oligonucleotid) und dazu komplementärem DNA-Aequorin-Konjugat auf SA-Festphase, doseresponse direkt proportional
- Testträger:: weiße Nunc MaxiSorb-Mikrotiterplatten, SA-beschichtet,
200 µl Gesamtreaktionsvolumen
- Probenverdünnung:: 1:2, d.h. 100 µl biotinylierte Fangsonde (= Probe) + 100 µl Konjugat
- Inkubationspuffer:: 25 mM HEPES pH 7.0, mit 150 mM KCl, 0.2% RSA, 10 mM EGTA, 0.05% Tween-20, 15 mM NaN₃ (= Puffer A) 125 mM Na-Phosphat pH 7.0, mit 1880 mM NaCl, 188 mM Na-Citrat, 50 mM MgCl₂, 40 mM EGTA, 4 mM EDTA, 0.125% RSA, 15 mM NaN₃ (= Puffer B) ==> Puffer A + Puffer B 1 + 1 gemischt
- Kalibratoren:: Konzentrationsreihe an Fangsonde (= Probe) in Inkubationspuffer
- Konjugat:: 20mer-DNA-Oligonucleotid-5'-MH:HS-Aequorin, ca. 2 x 10⁶ cps / Test
- Wascheinheit:: Source Instruments Plate Washer
- Triggerlösung:: 10 mM Tris-Puffer pH 7.5 mit 100 mM CaCl₂
- Meßeinheit:: Dynatech ML 3000 Plate Luminometer für MTP im enhanced flash mode, d.h. fixes Auswertungsintegral von 0 - 1.0 Sekunden nach Start der Triggerdosierung

Die Ergebnisse sind in Tabelle 11 zusammengefaßt

Mit dieser definierten 1:1-Konjugatstruktur gelingt *auf Anhieb* ein Hybridisierungsassay, der die gewünschten Merkmale wie breiter, dynamischer Meßbereich (4-5 Dekaden), gute Signal:Rausch-Verhältnisse und hohe analytische Sensitivität (im attomol-Bereich) aufweist. Diese guten Ergebnisse werden unabhängig von der Kopplungsposition (terminal, zentral) und der Kopplungschemie (terminales bzw. internucleosidisches Phosphoramidit, derivatisierte Nucleobase) erhalten.

Fazit: Die 1:1-Konjugatstruktur bestätigt sich in eindrucksvoller Weise als Garant auch für die Erstellung eines leistungsfähigen Hybridisierungsassays.

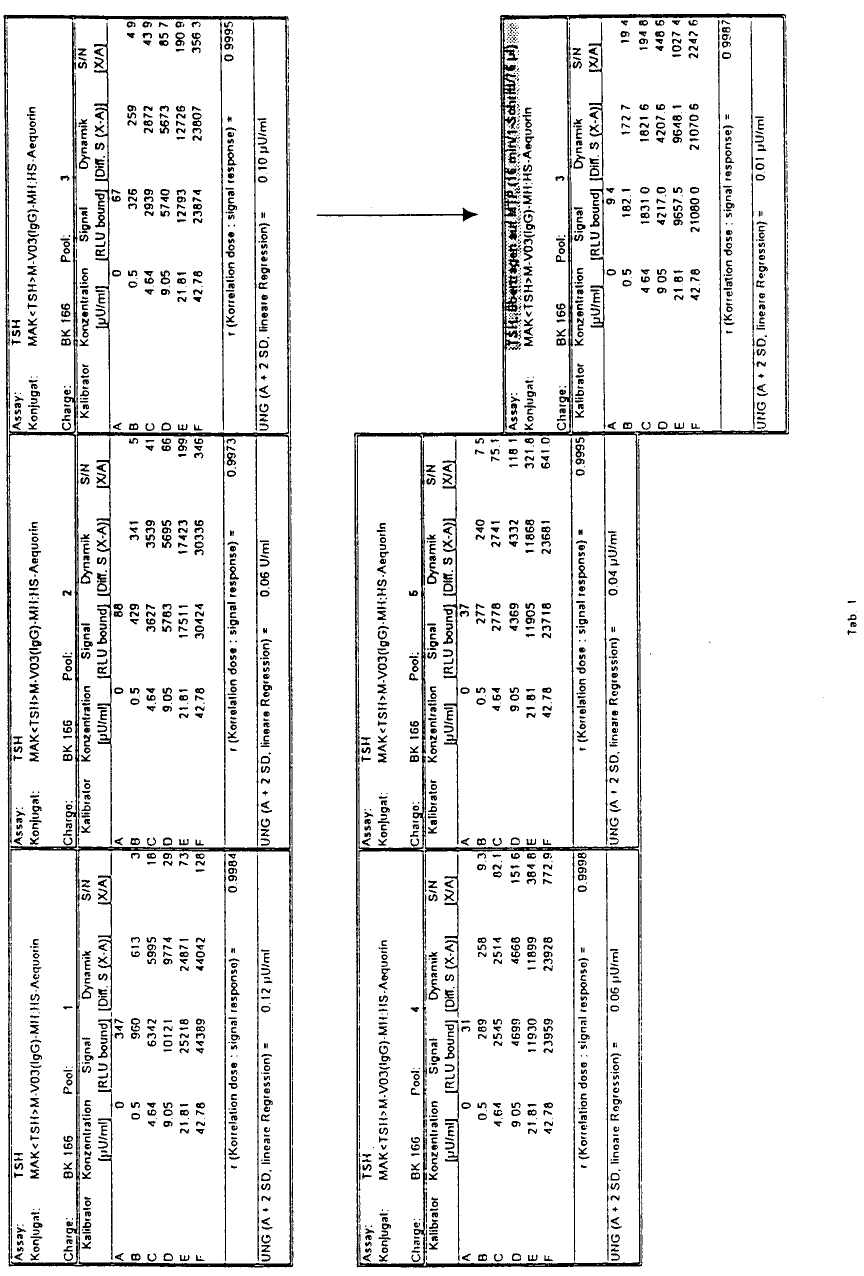

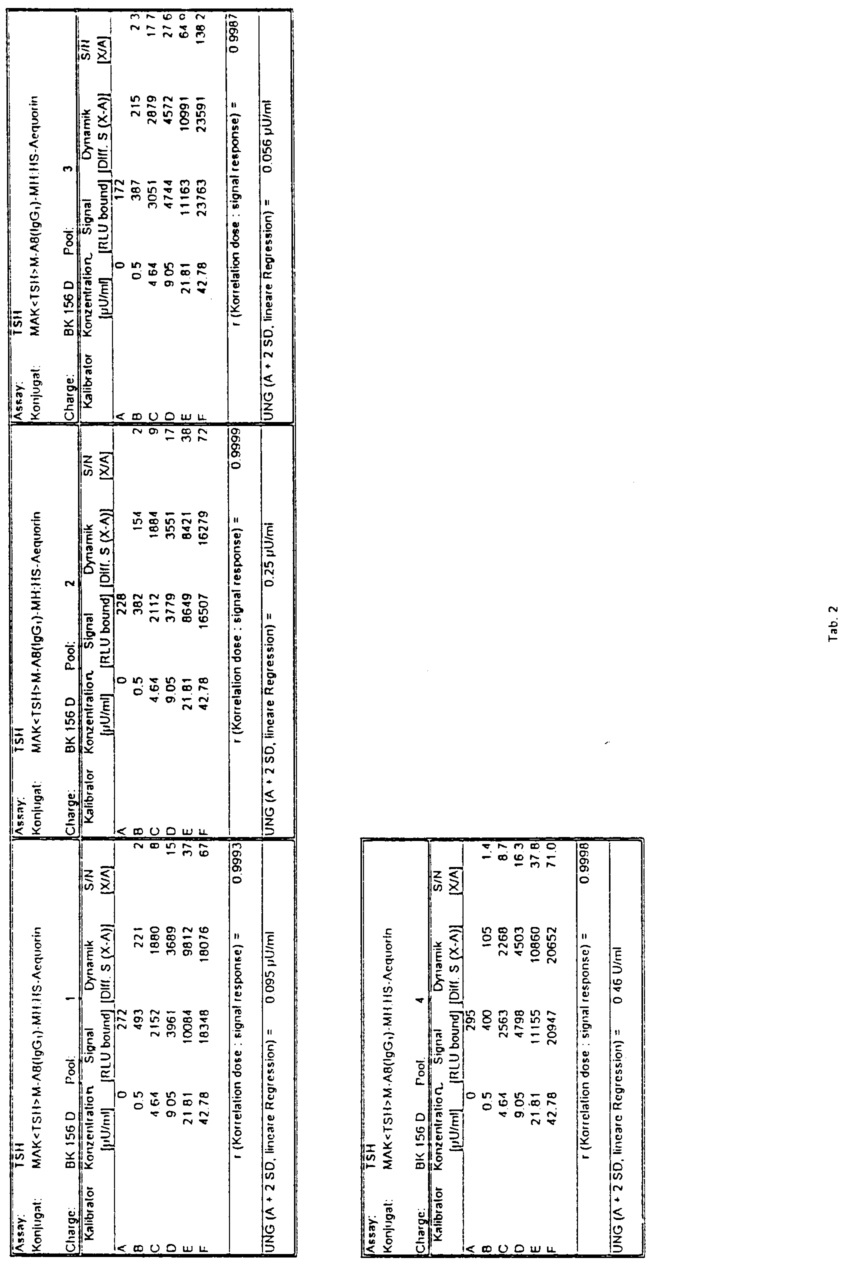

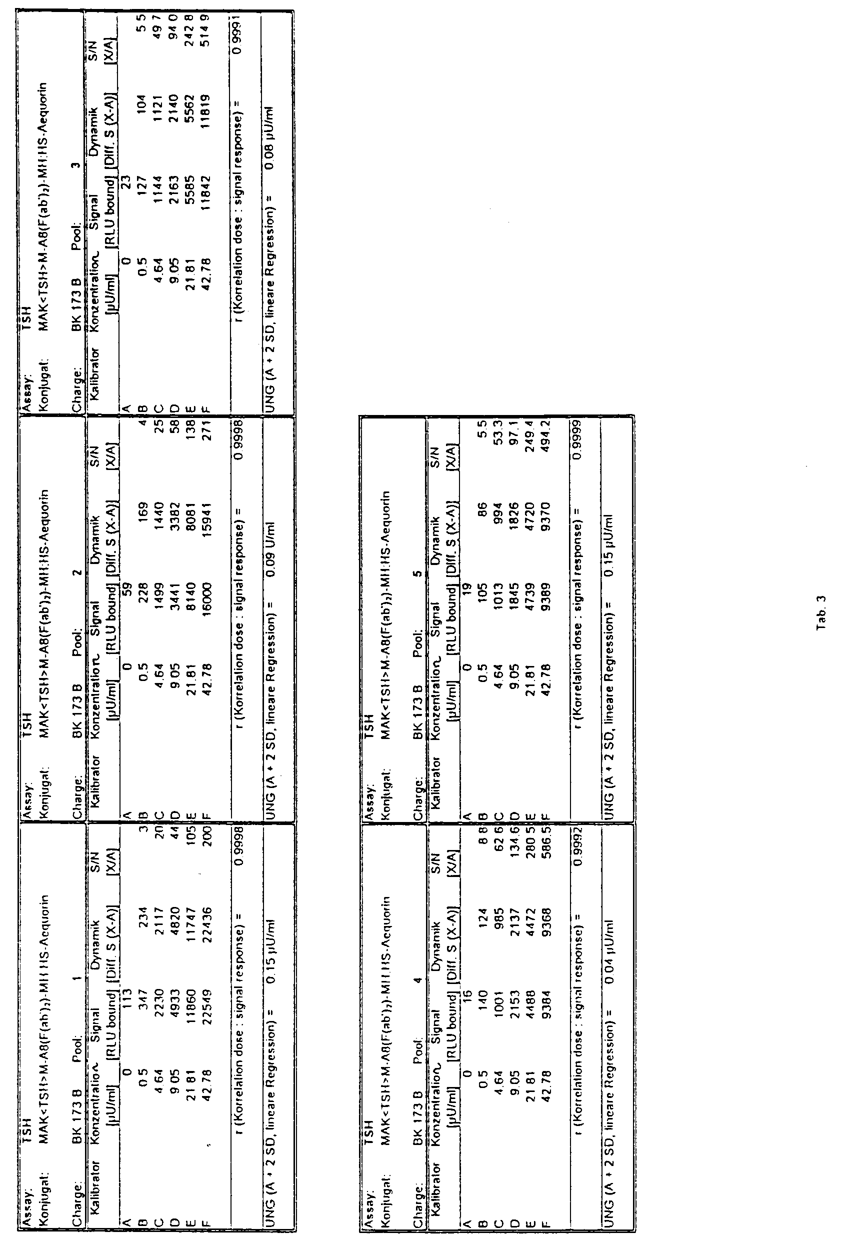

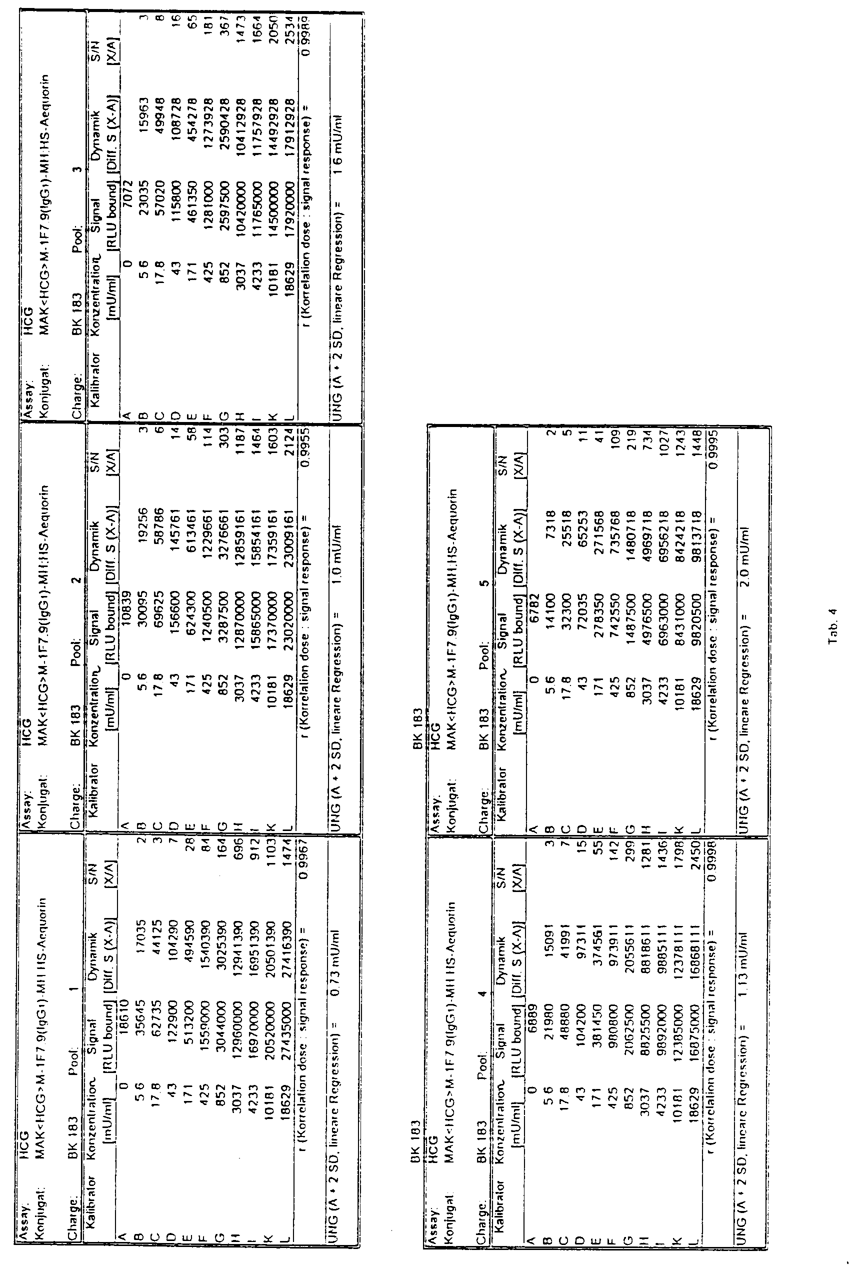

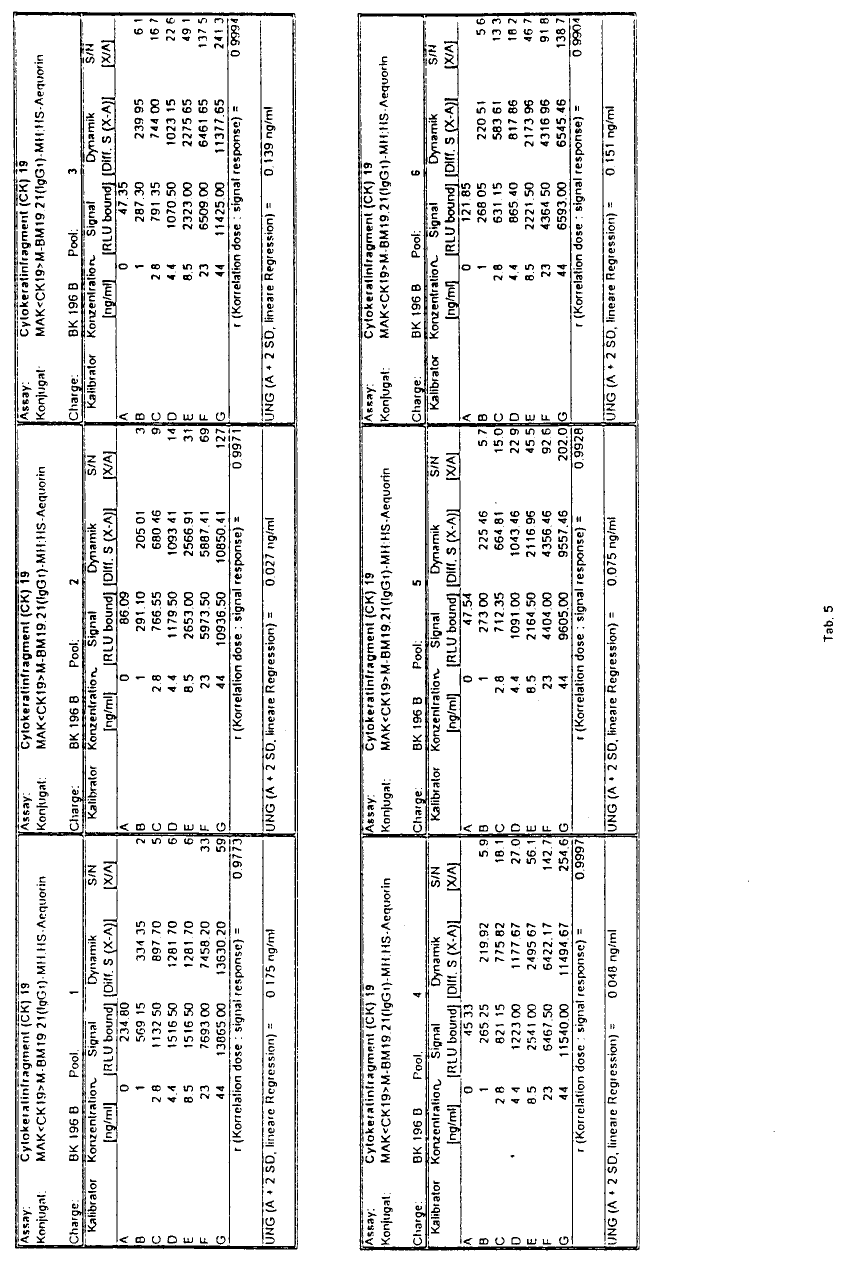

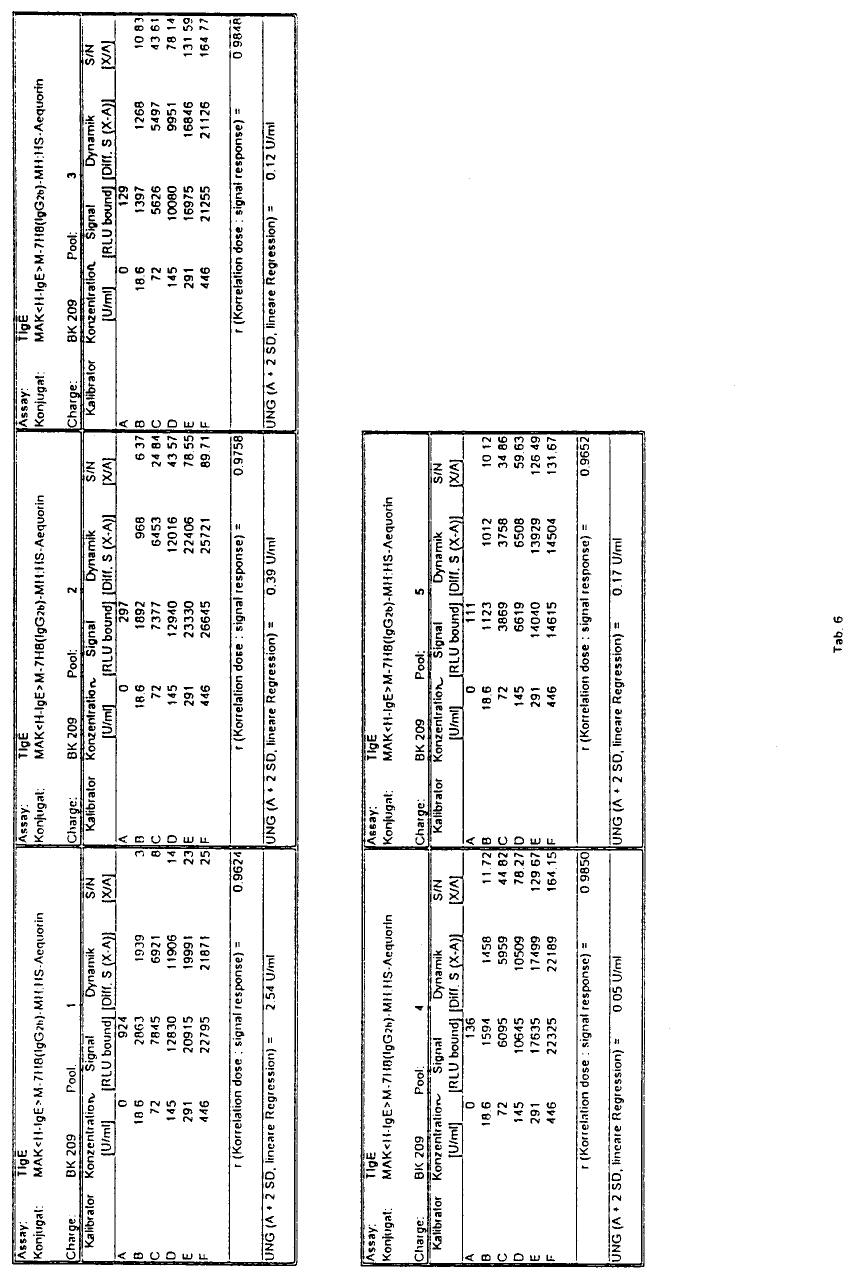

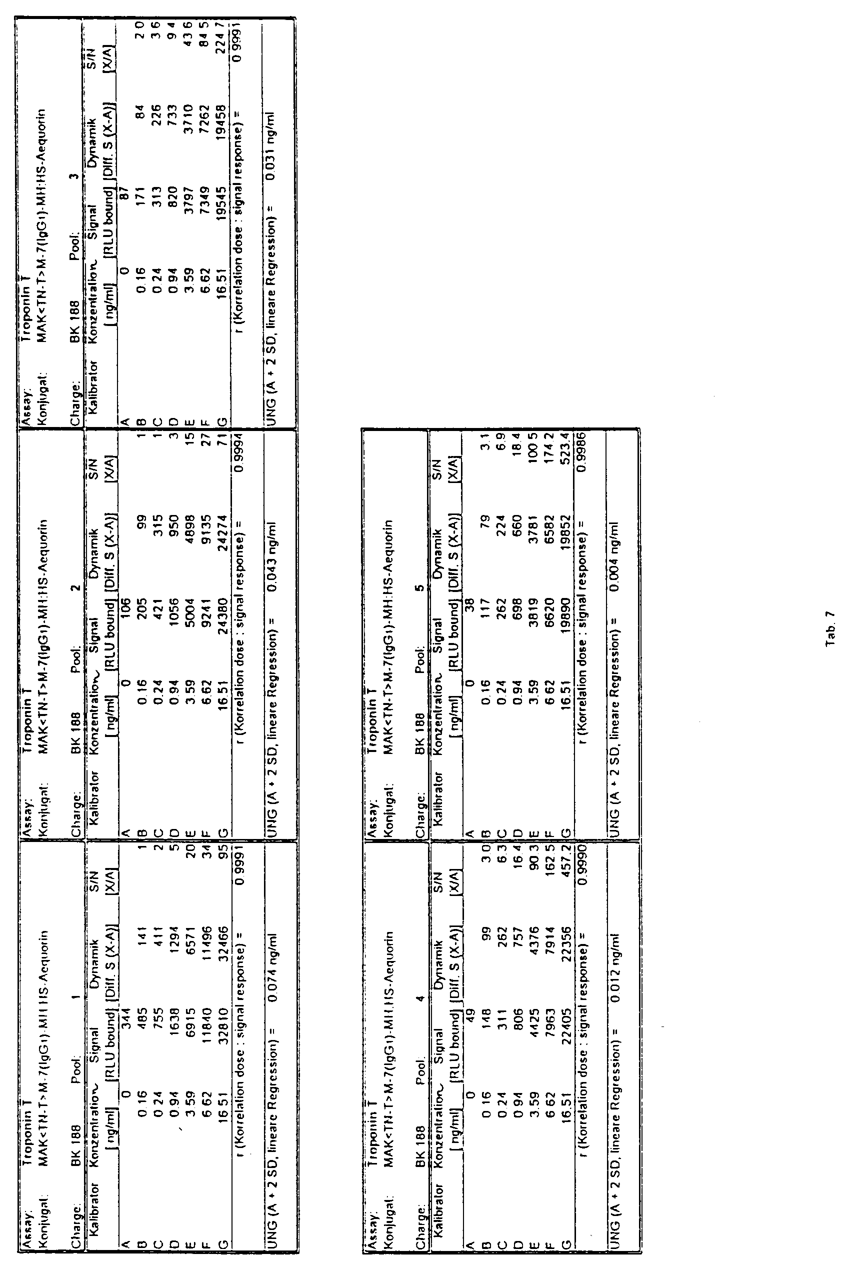

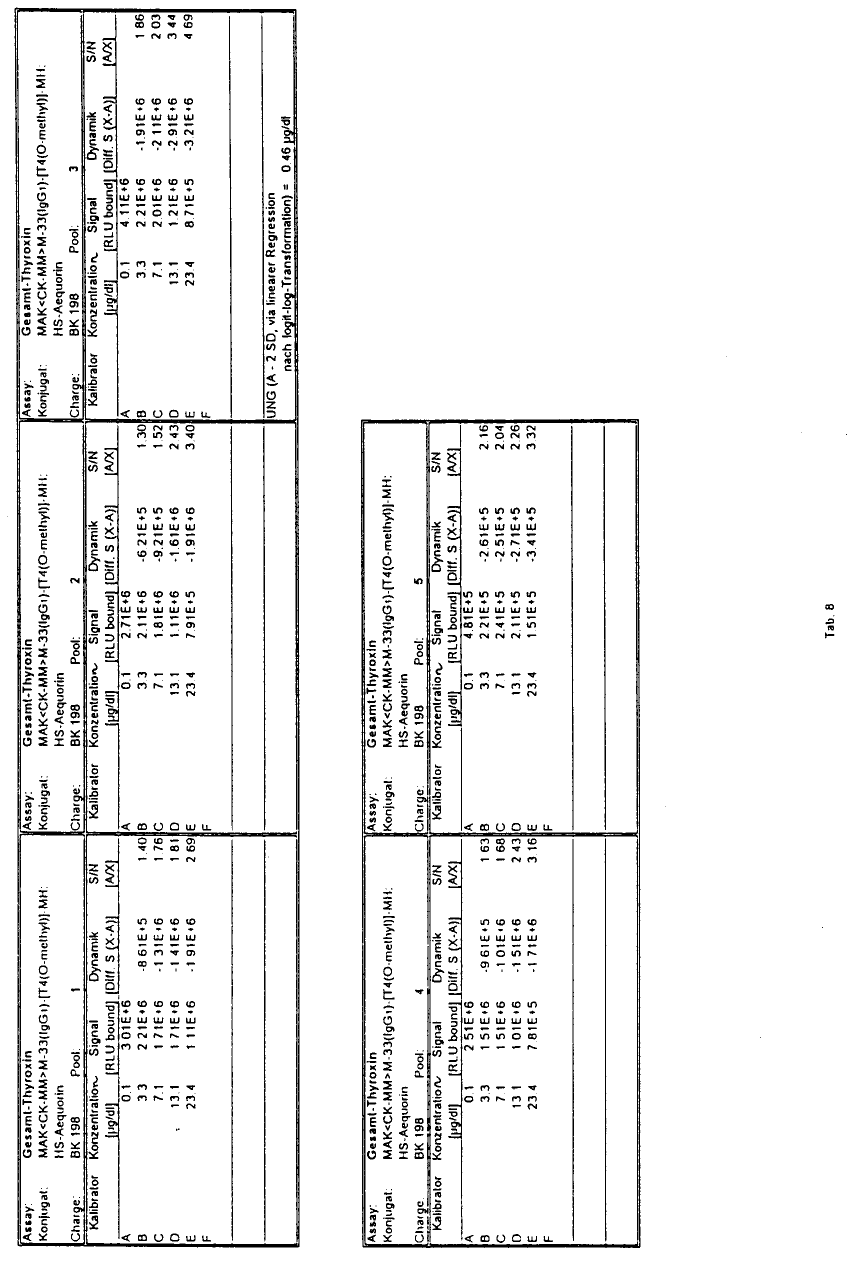

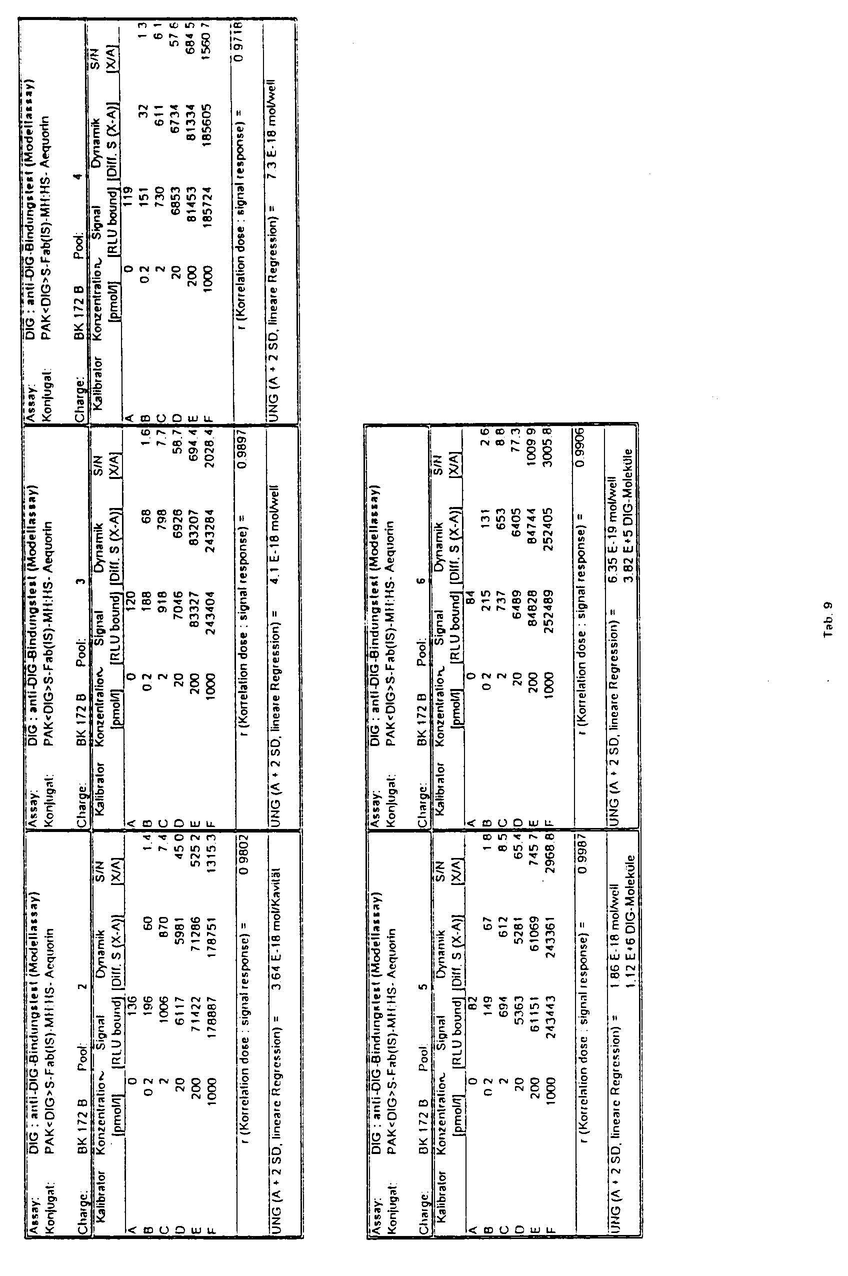

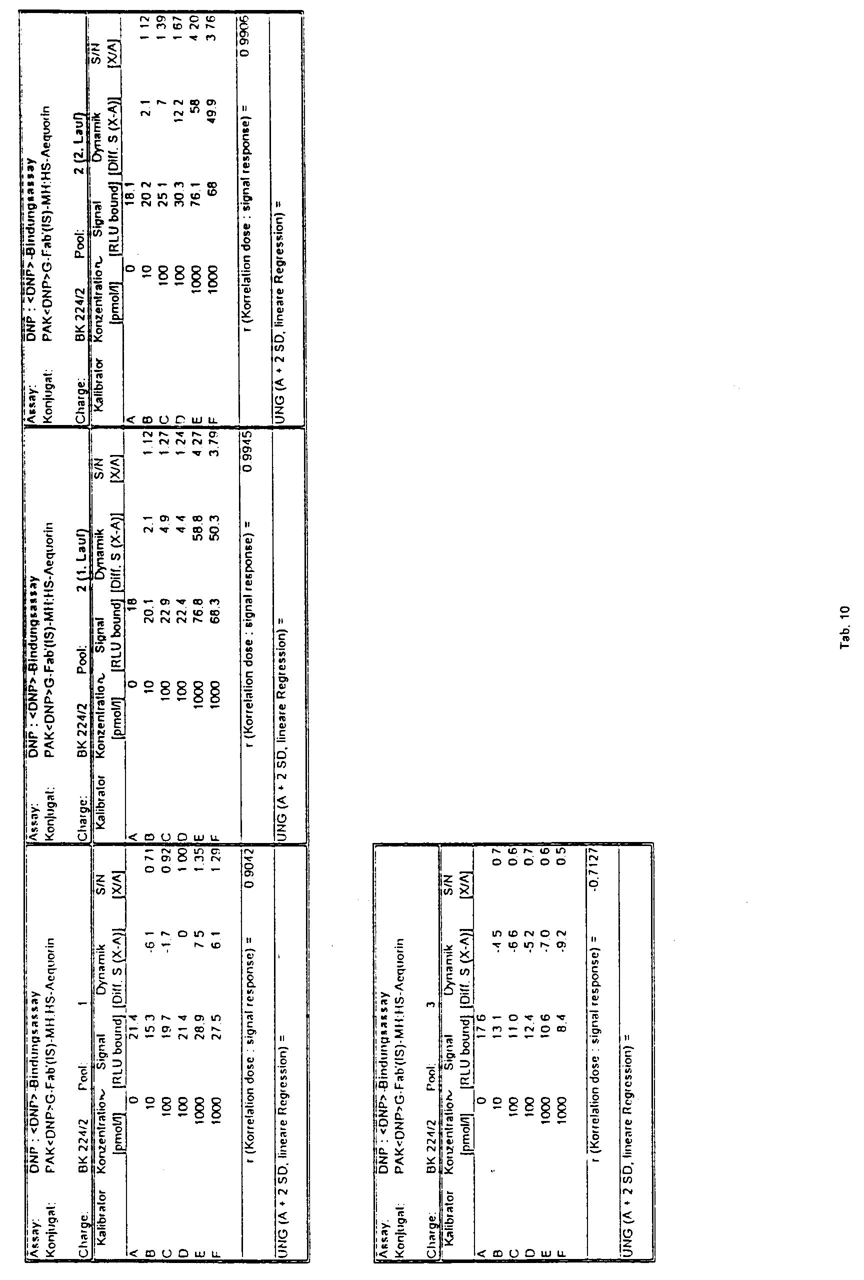

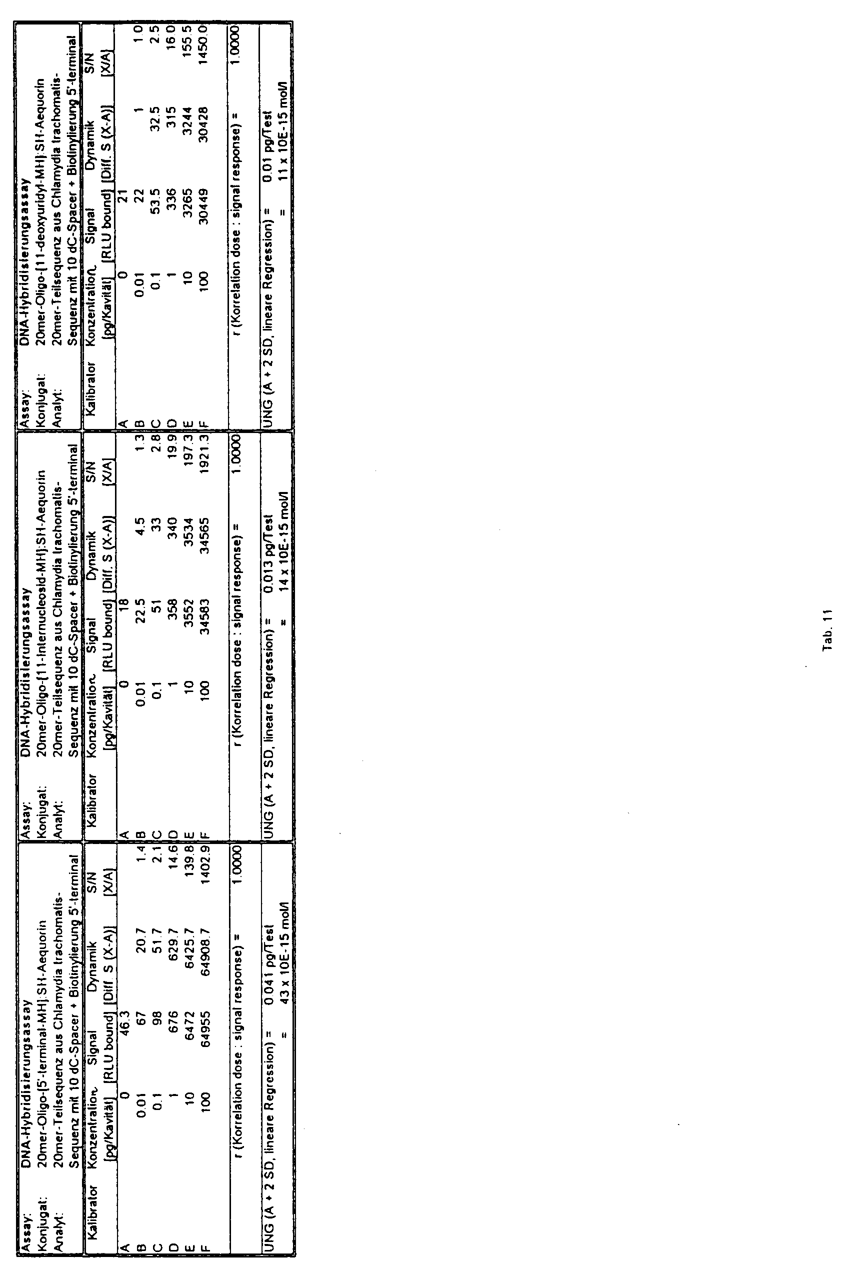

## Patentansprüche

1. Konjugatzusammensetzung aus einem Photoprotein und einem Bindepartner,
**dadurch gekennzeichnet,**
daß sie überwiegend Konjugatmoleküle enthält, in denen das Photoprotein und der Bindepartner in singulärer Anzahl kovalent miteinander verknüpft sind.

2. Konjugatzusammensetzung nach Anspruch 1,
**dadurch gekennzeichnet,**
daß das Molverhältnis von Photoprotein zu Bindepartner von 1 : 0,8 - 1 : 2 beträgt.

3. Konjugatzusammensetzung nach einem der vorhergehenden Ansprüche,
**dadurch gekennzeichnet,**
daß das Photoprotein ein Ca-aktivierbares Photoprotein ausgewählt aus der Gruppe umfassend Aequorin, Obelin, Clytin, Mitrocomin, Berovin, Mnemiopsin oder Thalassicolin ist.

4. Konjugatzusammensetzung nach einem der vorhergehenden Ansprüche,
**dadurch gekennzeichnet,**
daß das Photoprotein ein Sulfhydryl-aktiviertes Photoprotein ist und über die Sulfhydrylgruppe mit dem Bindepartner gekoppelt ist.

5. Konjugatzusammensetzung nach einem der vorhergehenden Ansprüche,
**dadurch gekennzeichnet,**
daß der Bindepartner eine biologische Substanz mit einem Molekulargewicht ≥ 4 kD ist.

6. Verfahren zur Herstellung einer Konjugatzusammensetzung nach einem der Ansprüche 1 - 5,
**dadurch gekennzeichnet,**
daß man ein Photoprotein und einen Bindepartner unter Bedinungen in Kontakt bringt, bei denen kovalente Kopplungsprodukte zwischen Photoprotein und Bindepartner gebildet werden und eine niedermolekulare Fraktion der Kopplungsprodukte von einer höhermolekularen Fraktion der Kopplungsprodukte und nichtreagierten Ausgangsmaterialien abtrennt.

7. Verfahren zum Nachweis eines Analyten in einer Probe durch Kontaktieren der Probe mit einem Analyt-spezifischen Nachweisreagenz,
**dadurch gekennzeichnet,**
daß man als Nachweisreagenz eine Konjugatzusammensetzung aus einem Photoprotein und einem Bindepartner nach einem der Ansprüche 1 - 5 verwendet.

8. Reagenz zur Durchführung eines Verfahrens nach Anspruch 7, umfassend eine Konjugatzusammensetzung aus einem Photoprotein und einem Bindepartner,
**dadurch gekennzeichnet,**
daß das Photoprotein und der Bindepartner überwiegend in singulärer Anzahl kovalent miteinander verknüpft sind.

9. Reagenzienkit zum Nachweis eines Analyten in einer Probe,
**dadurch gekennzeichnet,**
daß er neben anderen Testkomponenten ein Reagenz nach Anspruch 8 enthält.

10. Verwendung einer Konjugatzusammensetzung nach einem der Ansprüche 1 - 5 als Reagenz in einem Verfahren zum Nachweis eines Analyten.
